# FASCICULE DE BREVET EUROPEEN

(11) **EP 0 581 848 B1**
(45) Date de publication et mention de la délivrance du brevet: **02.07.2003**
(21) Numéro de dépôt: 92910423.0
(22) Date de dépôt: 24.04.1992
(51) Int. Cl.: C12N 15/11, A61K 31/70

(54) **OLIGONUCLEOTIDES FERMES, ANTISENS ET SENS, ET LEURS APPLICATIONS**
IN SICH GESCHLOSSENDE "SENS-" UND "ANTISENSE"-OLIGONUKLEOTIDE UND DEREN VERWENDUNGEN
CLOSED SENSE AND ANTISENSE OLIGONUCLEOTIDES AND USES THEREOF

(30) Priorité: 25.04.1991 FR 9105114
(43) Date de publication de la demande: 09.02.1994
(73) Titulaire: GENSET SA, F-75011 Paris (FR)
(72) Inventeur: BLUMENFELD, Marta, F-75013 Paris (FR); BRANDYS, Pascal, F-92150 Suresnes (FR); D'AURIOL, Luc, F-75019 Paris (FR); VASSEUR, Marc, F-75013 Paris (FR)
(74) Mandataire: Warcoin, Jacques
(86) Numéro de dépôt international: FR9200370
(87) Numéro de publication internationale: WO92019732

(56) Documents cités:
- EP-A- 0 321 201
- DE-A- 3 928 900
- Biochemistry, vol. 28, no. 1, 10 janvier 1989, (Easton, PA, US), D.A. ERIE et al.: "Melting behaviour of a covalently closed, single stranded, circular DNA", pages 268-273, voir le document en entier
- Nucleic Acids Research, vol. 11, no. 18, 24 septembre 1983, (Arlington, Virginia, US), T. YAGURA et al.: "Mechanism of stimulation by a specific protein factor of de novo DNA synthesis by mouse DNA replicase with fd phage single stranded circular DNA", pages 6369-6380, voir le document en entier
- Biochemical and Biophysical Research Communications, vol. 174, no. 3, 14 février 1991, (Duluth, Minnesota, US), N.N.L. SHEN et al.: "Introduction of single-stranded ADH genes into Drosophila results in tissue-specific expression", pages 1300-1305, voir le document en entier
- Chemical Abstracts, vol. 101, no. 17, 22 octobre 1984, (Columbus, Ohio, US), Y. OKADA: "Viroid, a replicating single-stranded circular RNA", page 149, colonne 1, abrégé no. 144891t, & GENDAI KAGAKU 1984, 160, 12-17, voir abrégé
- Nucleic Acids Research, vol. 17, no. 20, 25 octobre 1989, (Arlington, Virginia, US), D. MATTANOVICH et al.: "Stopping the DNA polymerase activity at a specific site with a dideoxyoligonucleotide: selective labelling of single stranded circular DNA", page 8483, voir le document en entier

## Description

La présente invention concerne des composés du type oligonucléotide ainsi que leurs applications.

Les oligonucléotides antisens sont de courtes molécules synthétique d'ADN -ou d'ARN- de séquence complémentaire à une séquence cible appartenant à un gène ou à un ARN messager dont on désire bloquer spécifiquement l'expression. Les oligonucléotides antisens peuvent être dirigés contre une séquence d'ARN messager, ou bien contre une séquence d'ADN. Les oligonucléotides antisens hybrident à la séquence dont ils sont complémentaires et peuvent ainsi bloquer l'expression de l'ARN messager portant cette séquence.
Le terme "oligonucléotide" est utilisé de façon générale pour désigner un polynucléotide en série ribo- ou désoxyribo-. Lorsqu'il sera question qu'une propriété particulière liée à l'utilisation d'une série désoxyribo- ou d'une série ribo-, on utilisera la dénomination complète oligodésoxyribonucléotide ou oligoribonucléotide. Un oligonucléotide peut être monocaténaire, c'est-à-dire ne comporter qu'un alignement de nucléotides, non appariés à un autre chaîne, ou bien être bicaténaire, c'est à dire comporter des nucléotides appariés à une autre chaîne polynucléotidique Deux oligonucléotides complémentaires forment une structure bicaténaire. Un oligonucléotide monocaténaire peut cependant présenter des régions bicaténaires par appariements intra-caténaire entre séquences complémentaires portées sur le même brin.
Le terme hybridation utilisé ici signifie la formation de liaisons hydrogènes entre des paires de bases complémentaires, la guanine et la cytosine formant trois liens hydrogènes, et l'adénine et la thymine en formant deux.

Les oligonucléotides antisens sont synthétisés par voie chimique et comportent fréquemment des modifications altérant le squelette même de la molécule ou portent des groupement réactifs additionnels, localisés à leurs extrémités. Ces modifications introduites dans les oligonucléotides antisens ont pour buts soit d'augmenter la résistance de ces molécules à la dégradation nucléolytique, soit de favoriser leurs interactions avec leurs cibles, soit de permettre des réactions de dégradation/modification spécifiques des cibles, ARN ou ADN, soit d'accroître leur pénétration intra-cellulaire.
Les oligonucléotides antisens sont sensibles aux dégradations nucléasiques, et principalement à l'action des exonucléases. Les nucléases se trouvent dans tous les compartiments -cellulaires et extra-cellulaires, en particulier dans le sérum- et provoquent une dégradation rapide de ces molécules. Une utilisation pharmacologique de molécules antisens implique la résolution de ces problèmes de dégradation afin d'arriver à une pharmacocinétique satisfaisante et donc à une pérennisation suffisante des effets de ces molécules. De nombreuses modifications chimiques permettent aux oligonucléotides antisens de devenir résistants aux nucléases. Certaines modifications affectent directement la structure ou la nature de la liaison phosphodiester (méthylphosphonates, phosphorothioates, oligonucléotides alpha, phosphoramidates pour citer quelques exemples), d'autre consistent en l'addition de groupements de blocage aux extrémités 3' et 5' des molécules (Perbost et al., 1989; Bertrand et al., 1989; Bazile et al., 1989; Andrus et al., 1989; Cazenave et al., 1989; Zon, 1988; Maher and Dolnick, 1988; Gagnor et al., 1987; Markus-Sekura, 1987).
Pour accroître l'efficacité des interactions entre un oligonucléotide et sa cible, on peut ajouter à une extrémité de l'oligonucléotide antisens un groupement intercalant (acridines par exemple). Enfin, on peut ajouter aux oligonucléotides antisens des groupements réactifs (agents alkylants, psoralènes, Fe-EDTA par exemple) capables de provoquer des coupures ou des altérations chimiques définitives au niveau de la cible (Sun et al, 1989; Helene, 1989; Durand et al., 1989; Sun et al., 1988; Helene and Thuong, 1988; Verspieren et al., 1987; Sun et al., 1987; Cazenave et al., 1988, 1987; Le Doan et al., 1987; Toulme et al., 1986; Vlassov et al., 1986).
Le dernier type de modification classique des oligonucléotides antisens consiste en l'addition de groupements modifiant la charge et/ou l'hydrophilie des molécules afin de faciliter leur passage trans-membranaire (Kabanov et al., 1990; Degols et al. 1989; Stevenson et al., 1989; Leonetti et al., 1988).
Toutes ces modifications peuvent évidemment être combinées entre elles.

Toutes les régions d'un ARN messager ne sont pas sensibles de la même façon aux effets d'un oligonucléotide antisens. Un ARN messager n'est pas une molécule linéaire figée, mais au contraire une molécule comportant de nombreuses structurations secondaires (hybridations intra-moléculaires complexes), tertiaires (repliements et conformations particulières, pseudo noeuds), et interagissant avec des nucléoprotéines structurales et fonctionnelles (protéines basiques, complexes d'épissage, de poly-adénylation, de capping, complexe de traduction par exemple). La disponibilité effective et l'accessibilité des différents régions d'un ARN messager va dépendre de leurs engagements dans ces structurations. Corrélativement, l'efficacité d'un agent inhibiteur interagissant avec telle ou telle séquence va dépendre également de l'engagement de cette séquences dans une fonction particulière. Les régions cibles pour les molécules antisens doivent être accessibles à l'oligonucléotide.
L'utilisation de logiciels de prédiction de structures secondaires permet de prévoir des degrés d'accessibilité théoriques et donc d'orienter le choix de cibles pour des oligonucléotide antisens. Globalement, les régions les plus utilisées comme cibles sont les sites de démarrage de la traduction (région de l'AUG initiateur) ainsi que les sites d'épissage (jonctions SD/SA). De nombreuses autres séquences n'ayant pas de fonctionnalités particulières et non engagées dans des appariement intra-moléculaires se sont avérées également efficaces comme cible pour des oligonucléotides antisens (voir les exemples cités plus loin).

Les oligodésoxyribonucléotides antisens peuvent également être dirigés contre certaines régions d'ADN bicaténaire (séquences homopurines/homopyrimidines ou riches en purines/pyrimidines) et former ainsi des triples hélices (Perroualt et al., 1990; François et al.(A), 1989; François et al.(B), 1989; François et al.(C), 1989; Wang et al., 1989; Maher et al., 1989; Sun et al., 1989; Boidot-Forget et al., 1988; Moser and Dervan, 1987; Dervan, 1986). Des oligonucléotides dirigées ainsi contre l'ADN ont été appelés "anti-gène" ou encore "anti-code". La formation d'une triple hélice, au niveau d'une séquence particulière, peut bloquer la fixation de protéines intervenant dans l'expression d'un gène et/ou permettre d'introduire des dommages irréversibles dans l'ADN si l'oligonucléotide considéré possède un groupement réactif particulier. De tels oligonucléotides antisens peuvent devenir de véritables endonucléases de restriction artificielles, dirigées à la demande contre des séquences spécifiques.

L'hybridation entre un oligonucléotide antisens et un ARN messager cible peut en bloquer l'expression de plusieurs manières, soit de façon stérique, soit de façon pseudo-catalytique (Gagner et al., 1989; Jesus et al., 1988; Markus-Sekura, 1987):
- l'interaction entre l'ARN messager et un oligonucléotide antisens complémentaire peut créer une barrière physique interdisant la fixation et/ou la progression de protéines ou de complexes protéiques nécessaire à la traduction, à la maturation, à la stabilisation ou au transport de l'ARN messager. Ce blocage physique aboutira finalement à une inhibition de l'expression de l'ARN messager ciblé.
- l'hybridation entre un ARN messager et un oligodésoxyribonucléotide antisens va créer un substrat pour la RNase H, enzyme présent dans toutes les cellules eucaryotes. La RNase H est un enzyme qui dégrade spécifiquement l'ARN lorsqu'il est hybridé à de l'ADN. L'hybridation d'un oligonucléotide antisens à un ARN cible aboutira donc à la coupure de cet ARN cible à l'emplacement de cette hybridation, et donc à son inactivation définitive.
- Par ailleurs, comme indiqué plus haut, les oligonucléotides antisens peuvent comporter des groupement réactifs capables de produire directement des dommages irréversibles dans la molécules d'ARN cible.

En ce qui concerne les oligonucléotides antisens dirigés contre l'ADN ils peuvent agir soit en inhibant la fixation d'une protéine de régulation indispensable à l'expression du gène ciblé (facteur de transcription par exemple), soit en introduisant des dommages irréversibles (coupures, cross-links) dans la molécule d'ADN, la rendant inapte, localement, pour l'expression génétique.

Les ribozymes sont des molécules d'ARN douées d'activités enzymatiques, capables en particulier de provoquer des coupures endonucléasiques dans des ARNs cibles. Un ribozyme peut être considéré comme un oligonucléotide antisens particulier, doué d'une activité catalytique endonucléasique naturelle (Vasseur, 1990; Symons, 1989; Jeffrie and Symons, 1989; Haseloff and Gerlach, 1988; Uhlenbeck, 1987; Symons et al., 1987). Typiquement, un ribozyme est constitué de deux parties, d'une part il comporte une séquence complémentaire à la séquence cible que l'on désire couper, et d'autre part, une séquence catalytique, faisant office de groupement réactif (Fedor and Uhlenbeck, 1990; Uhlenbeck et al; 1989; Sheldon and Symons, 1989; Sampson et al., 1987). Il est possible actuellement, en utilisant un site actif consensus déduit de la séquence de ribozymes viraux, de couper théoriquement n'importe quel ARN messager, en une position déterminée à l'avance (Haseloff and Gerlach, 1988; Uhlenbeck, 1987). Les ribozymes rencontrent les mêmes problèmes d'utilisation que les oligonucléotides antisens classiques, en particulier en ce qui concerne les phénomènes de dégradation, les ARNs étant encore plus sensibles à la dégradation nucléolytique que les ADNs.

Les oligonucléotides antisens permettent de bloquer spécifiquement l'expression d'ARN messagers cellulaires, par exemple de messagers de type oncogénique, (Tortora et al., 1990; Chang et al., 1989; Anfossi et al., 1989; Zheng et al., 1989; Shuttleworth et al., 1988; Cope and Wille, 1989; Cazenave et al., 1989) et de nombreux différents types d'ARN messager viraux, provenant de virus aussi variés que le VSV (Degols et al., 1989; Leonetti et al., 1989), le SV40 (Westermann, et al., 1989), les virus influenza (Kabanov et al., 1990; Zerial et al., 1987), le virus de l'encéphalomyocardite (Sankar et al., 1989), l'adénovirus (Miroshnichenko et al., 1989), le HSV (Gao et al., 1988) et le HIV (Matzukura et al., 1989; Stevenson et al., 1989; Matzukura et al., 1988; Goodchild et al., 1988).
Avec des ribozymes, on peut cliver *in vivo* l'ARN messager codant, pour le gène marqueur CAT (Cameron and Jennings, 1989), inhiber le processus de maturation de l'ARN messager d'histone (Cotten et al.1989; Cotten and Birnstiel, 1989) ou protéger partiellement la cellule contre l'infection par HIV-1 (Sarver et al., 1990).

Les oligonucléotides peuvent également être utilisés dans le cadre de stratégies de type "sens". Cette approche consiste à utiliser un oligonucléotide en série désoxyribo- ou ribo-, monocaténaire ou bicaténaire, de séquence spécifique, comme agent de fixation d'une protéine présentant une affinité pour cette séquence et dont on désire diminuer la concentration efficace, par compétition, à l'intérieur des cellules. On peut envisager ainsi d'utiliser des oligonucléotides interagissant avec des facteurs de transcription, des facteurs d'encapsidations viraux, des facteurs de régulation de la traduction, etc... Cette approche n'est pas encore exploité comme l'est la stratégie antisens plus classique. Dans ce cas, le problème de la stabilité des oligonucléotides est également un facteur important critique pour l'efficacité et la pérennité de leur action. L'utilisation d'oligonucléotides modifiés pour une telle approche peut se heurter à des problèmes structuraux de reconnaissance par les protéines. La possibilité de disposer d'oligonucléotides naturels stables dans le sérum et les cellules permettrait d'envisager le développement de nouvelle voies thérapeutiques ciblées en particulier sur les facteurs de régulations à affinité pour les acides nucléiques.

Les oligonucléotides antisens, et sens, sont donc des agents pharmacologiques potentiels, puissants et hautement spécifiques, permettant d'inhiber l'expression de messagers codant pour des produits exerçant des effets pathogènes.
L'utilisation thérapeutique des oligonucléotides se heurte cependant à plusieurs problèmes de type physiologiques, en particulier celui de la délivrance intracellulaire de ces molécules et celui de leur sensibilité à la dégradation nucléolytique. L'utilisation de dérivés modifiés permet de surmonter le problème de la sensibilité aux nucléases, mais introduit un nouveau problème, celui de la toxicité éventuelle des modifications chimiques introduites dans la molécule.
L'utilisation des oligonucléotides antisens modifiés pose en effet des problèmes de nature toxicologiques. Si certaines des modifications sont réputées plutôt neutres, la plupart ne sont pas dénuées de toxicité potentielle. Des oligonucléotides antisens modifiés chimiquement peuvent présenter une toxicité à plusieurs niveau, soit directement par des effets de la molécule entière, soit indirectement *via* les effets des produits de dégradation. Des nucléotides porteurs de modifications chimiques, et présents dans une cellule à une concentration élevée peuvent ainsi présenter une toxicité -et plus particulièrement une génotoxicité- non négligeable au plan pharmacologique.
Par exemple, de nombreux problèmes soulevés par l'emploi d'oligonucléotides antisens modifiés, en particulier des effets anti-viraux non séquence-spécifiques, semblent bien être dus à la nature de certaines des modifications chimiques introduites dans les oligonucléotides antisens pour les rendre résistant aux nucléases.

Au plan toxicologique, il est donc évident que mons on modifie la structure naturelle de l'oligonucléotide, moins on risque d'être confronté à des problèmes pharmacologiques. Une molécule naturelle d'ADN ou d'ARN, ainsi que ses produits de dégradation, ne pose pas ou peu de problème de toxicologie et de pharmacocinétique, ce qui n'est pas le cas d'une structure modifiée pouvant donner après métabolisation des dérivés multiples et potentiellement toxiques.

Il serait donc avantageux de pouvoir disposer d'oligonucléotides naturels, ne comportant que des désoxy ou ribonucléotides normaux, reliés entre-eux par une liaison phosphodiester normale, mais présentant cependant une résistance à la dégradation.

L'invention présentée ici a pour objet un nouveau type structural d'oligonucléotide antisens, ou sens, résistant aux exonucléases sans intervention de modifications chimiques stabilisantes. Les oligonucléotides faisant l'objet de l'invention ont la particularité de présenter une structure fermée, n'offrant pas d'extrémité disponible à la dégradation exonucléasique.

De tels oligonucléotides peuvent être utilisés dans leur état naturel mais peuvent cependant comporter également des nucléotides modifiés, des groupements réactifs, ou être associés physiquement à d'autres molécules ou macromolécules dans le but de renforcer leur efficacité d'inhibition, leur pénétration, leur affinité pour leurs cibles, leur ciblage cellulaire ou intra-cellulaire, ou pour optimiser toute autre propriété.

### II. DESCRIPTION DE L'INVENTION

Dans les cellules, et plus encore dans un organisme, dans la circulation sanguine par exemple, les oligonucléotides antisens naturels sont sensibles aux nucléases. Les nucléases sont des enzymes de dégradation capables de couper les liaisons phosphodiester de l'ADN ou de l'ARN, soit en introduisant des coupures internes sur des molécules mono- ou bicaténaire, soit en attaquant ces molécules à partir de leurs extrémités. Les enzymes attaquent de façon internes sont appelées les endonucléases et celles attaquant par les extrémités sont appelés axonucléases.

La stabilité des oligonucléotides antisens -donc leur efficacité- peut être considérablement augmentée en introduisant diverses modifications chimiques les rendant résistant à la dégradation comme décrit ci-dessus.
Il est établi que ce sont les exonucléases qui sont la principale cause de dégradation des oligonucléotides antisens dans le sérum et dans la cellule. Plus particulièrement, il semble que les exonucléases s'attaquant à l'extrémité 3'OH soient surtout à incriminer dans ce phénomène.
Des modifications apportées à la structure des extrémités des oligonucléotides antisens peuvent les protéger, bloquer les activités des exonucléases, et conférer aux oligonucléotides une stabilisation accrue.

L'invention décrite ici est fondée sur l'idée nouvelle que des oligonucléotides fermés, ne comportant pas d'extrémités libres, seront donc, par définition, résistants à ce type de dégradation. Des oligonucléotides fermés, circulaires par exemple, ne présentent pas de substrat accessible aux exonucléases 3' ou 5' et sont donc ainsi stabilisés.

Plus particulièrement, l'invention concerne donc un agent antisens ou sens du type oligonucléotide, caractérisé en ce qu'il est constitué d'une ou plusieurs séquence(s) d'oligonucléotides monocaténaires dont les extrémités sont reliées entre elles par des liens covalents pour former au moins partiellement une structure monocaténaire fermée.

Ces agents sont parfois dénommés ci-après oligonucléotides fermés ou oligonucléotides circulaires, dans la mesure où ce type de composé présente de préférence une majorité de nucléotides par rapport aux structures non nucléotidiques.

La définition du terme oligonucléotide a été donnée précédemment et incorpore aussi bien la série ribo- que désoxyribo- naturelle, de même que les modifications de ces bases qui seront globalement dénommées ci-après non-naturelles et qui ont également été évoquées précédemment.

Le lien covalent peut être une structure covalente non nucléotidique protéique, lipidique, osidique et/ou une structure mixte, comme cela sera explicité ci-après. On préfère toutefois utiliser une structure covalente nucléotidique, c'est à dire une liaison phosphodiester.

L'invention concerne des oligonucléotides fermés, circulaires par exemple, n'ayant pas d'extrémités libres, mais composés par une suite de bases nucléotidiques reliées entre elles par tout types de liaisons, et préférentiellement par des liaisons de type phosphodiester. Ces bases pourront être associées entre elles par des liaisons telles que la distance entre ces bases sera d'environ 3 A à 4 A ce qui est la distance que l'on trouve dans les molécules naturelles d'ADN ou d'ARN lorsque les liaisons internucléotidiques sont assurées par des groupements phosphodiesters. Les oligonucléotides fermés, circulaires par exemple, seront composés avantageusement de nucléotides naturels liés préférentiellement entre-eux par des liaisons phosphodiesters non modifiées, mais pourront également comporter des nucléotides modifiés et/ou des liaisons modifiées respectant les distances entre bases et permettant les rotations axiales hélicales caractéristiques des conformations des acides nucléiques. Les oligonucléotides fermés seront donc composés avantageusement des bases A, T, G, C, ou U, sous leurs formes désoxy- ou ribonucléotidiques. Les oligonucléotides fermé pourront donc être soit des oligodésoxyribonucléotides, soit des oligoribonucléotides, soit des molécules mixtes comportant des désoxyribonucléotides et des ribonucléotides.

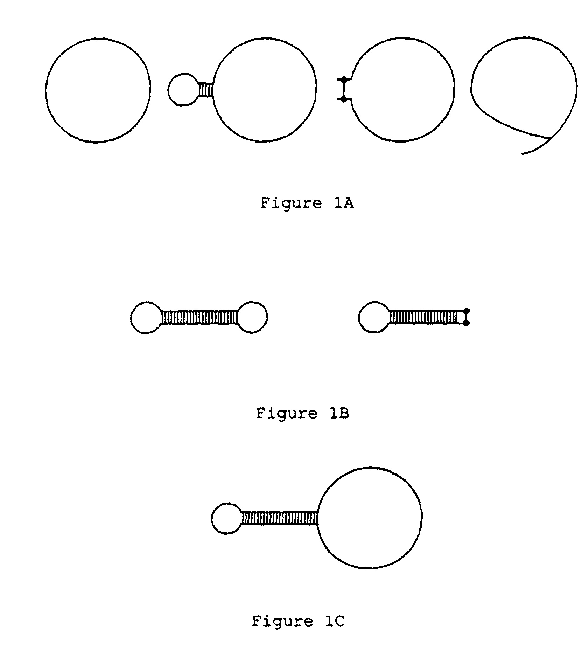

Sont donc comprises dans l'invention, toute molécule d'ADN, ou d'ARN, -ou mixte ADN/ARN-, monocaténaire, fermée, circulaire ou possédant une partie circulaire, obtenue de façon biologique, chimique, ou par des procédés associant les techniques de la chimie de synthèse à celles de la biologie et de la biochimie, présentant une résistance aux exonucléases supérieure à celle d'un oligonucléotide de même séquence mais complètement linéaire, ne présentant pas de structure fermée.
Des exemples d'oligonucléotides fermés sont présentés dans les figures 1A à 1C.

La figure 1A présente des exemples de structure d'oligonucléotides antisens fermés. La figure 1B présente des exemples de structure d'oligonucléotides sens fermés. La figure 1C présente une molécule mixte pouvant exercer un effet sens et antisens.

Le nombre de nucléotides unitaires composant les oligonucléotides fermés peut varier dans de larges proportions notamment entre 10 et 200, mais ce nombre sera avantageusement compris, à titre indicatif, entre une vingtaine et une cinquantaine de nucléotides, selon la structure de fermeture (circulaire, structure en lasso, en ballon, structure bicaténarisée, etc... voir plus loin les descriptions des différentes structures), les utilisations (molécule antisens anti-ARN, molécule antisens anti-ADN, molécule sens anti-protéique), le type de l'oligonucléotide -désoxy ou ribonucléotidique- (antisens simple ou antisens ribozymique, etc...) et les cibles considérées (ARNmessager, ARN prémessager, structure secondaire particulière, etc..).

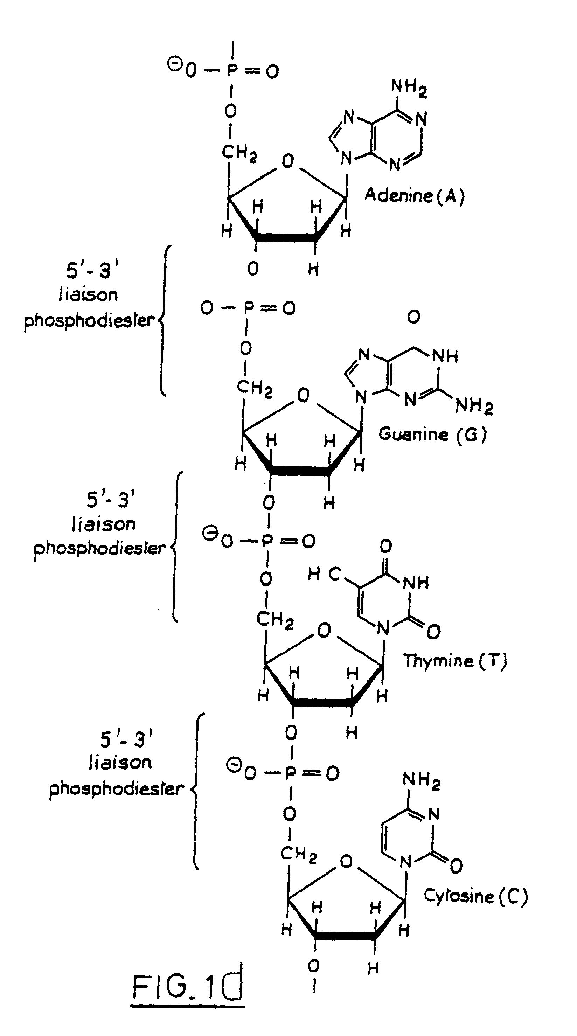

Les oligonucléotides fermés faisant l'objet de la présente invention sont préférentiellement composés par une séquence de bases comportant de l'adénine (A), de la guanine (G), de la cytosine (C), de la thymine (T) et de l'uracile (U), de formules générales présentées en figure 1D.

Les oligonucléotides fermés pourront également comporter des nucléotides rares (Inosine, I, ou rI par exemple) ou des nucléotides modifiés, soit en série désoxyribo- soit en série ribo-.

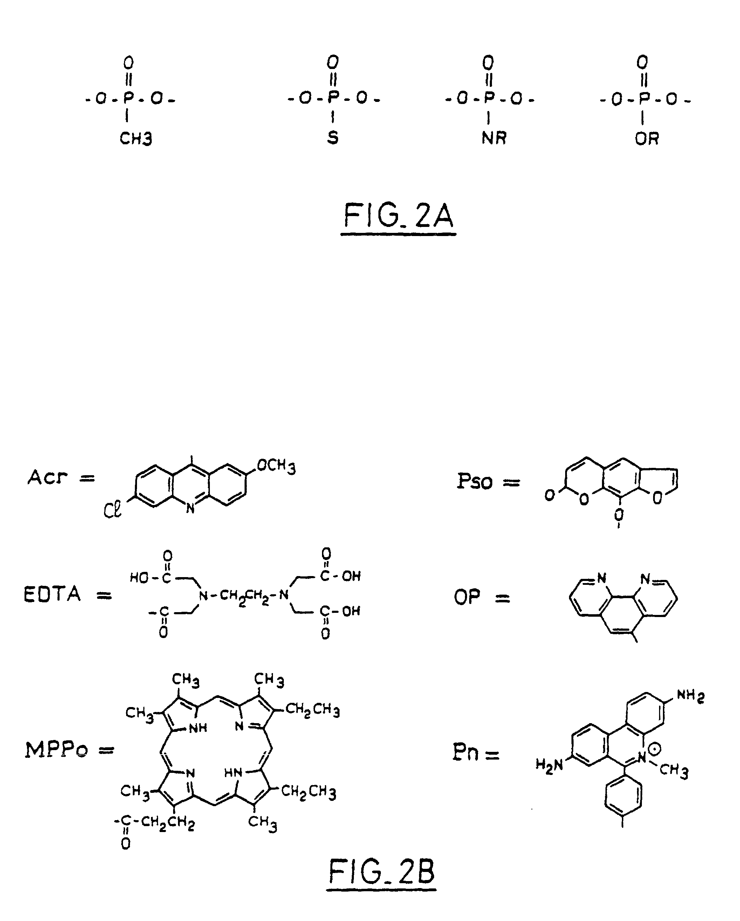

Les oligonucléotides fermés pourront comporter des nucléotides modifiés au niveau de la liaison phosphodiester, par exemple, selon les quelques formules présentées en figure 2. Par exemple, les oligonucléotides fermés pourront comporter un ou plusieurs des groupements bien connus que sont les phosphorothioates, les méthylphosphonates, les phosphorodithioates, les phosphoselonates, les phosphoroamidates, les alkyl-phosphotriesters. Notons cependant que les oligonucléotides antisens fermés comprendront préférentiellement des nucléotides naturels, reliés entres-eux par des liaisons phosphodiesters non modifiées.

Les oligonucléotides fermés pourront comporter des nucléotides réactifs, capables d'établir des liens avec la séquence de la molécule cible complémentaire à l'oligonucléotide.

Ainsi, les oligonucléotides fermés pourront porter des groupements réactifs greffés sur les nucléotides, comme par exemple des groupements psoralènes, ou d'autres agents de pontage ou agents intercalant pouvant réagir avec la séquence de la molécule cible complémentaire à l'oligonucléotide (voir les quelques exemples non-exhaustifs présentés en figure 2).

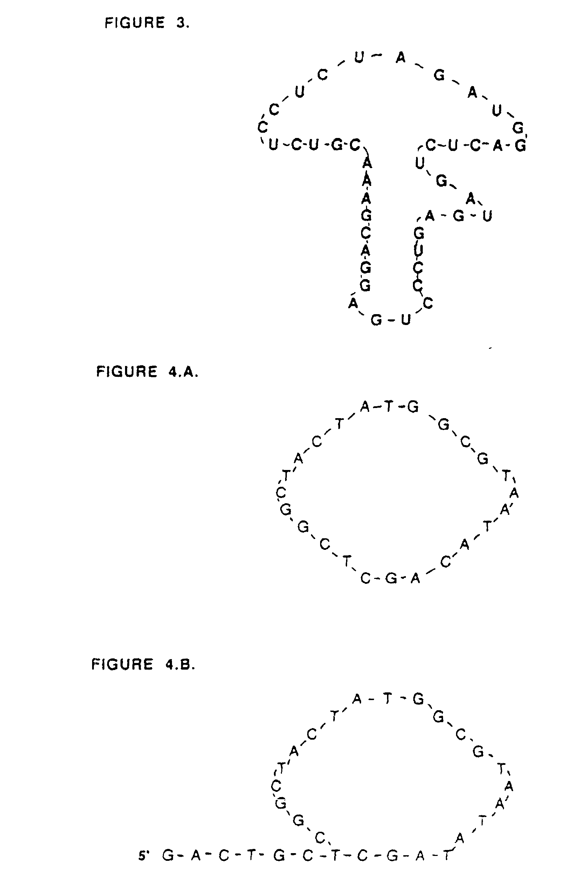

Les oligonucléotides antisens fermés pourront également inclure, parmi les nucléotides faisant partie de la structure fermée, une séquence d'ARN présentant une activité catalytique. Ces oligonucléotides circulaires seront ainsi des ribozymes circulaires, aux extrémités jointes, comportant, en plus de la séquence catalytique pouvant être toute séquence d'ARN capable de provoquer une coupure ou un modification dans une séquence d'ARN cible, une séquence de type antisens, complémentaire à la séquence ciblée, et pouvant être constituée soit d'ARN, soit d'ADN soit d'un mélange ADN/ARN (figure 3).

Font également partie de l'invention des oligonucléotides fermés couplés à des molécules permettant d'accroître leur pénétration intra-cellulaire, et en particulier des groupement lipophiles, des polypeptides ou des protéines.

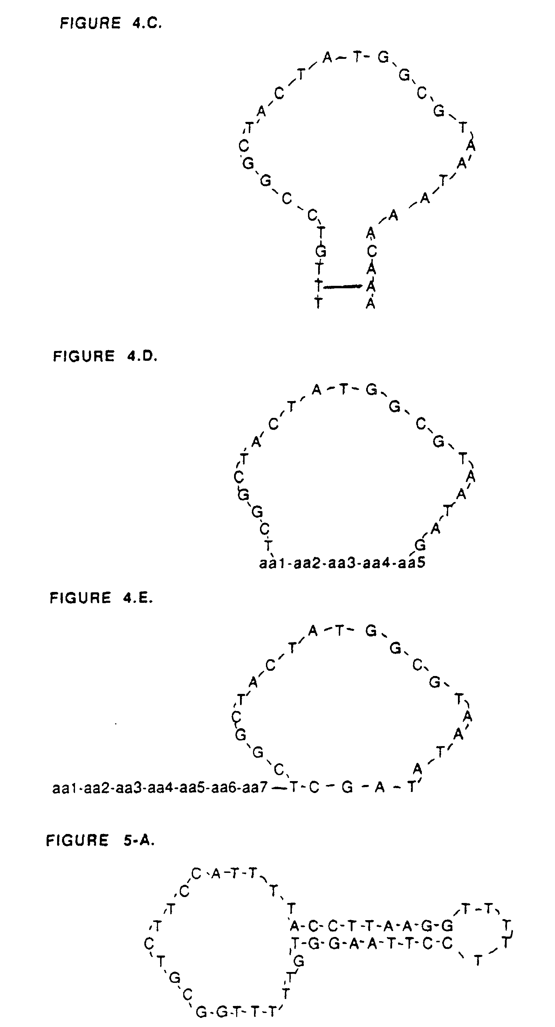

Les oligonucléotides fermés présentent la caractéristique principale de ne pas offrir de substrat aux exonucléases 3' et 5'. Pour acquérir cette propriété, la structure la plus simple est un oligonucléotide circulaire, formée d'une succession de nucléotides reliés entre eux par des liaisons phosphodiesters comme schématisé plus haut, et présentant ou non des appariements intracaténaires (figures 1A à 1C et figure 4).

D'autres structures de molécules fermées par une liaison non phosphodiester 3'-5' peuvent également être synthétisées et présenter une résistance partielle ou totale aux exonucléases.

Font également partie de l'invention des molécules en lasso composées de résidus désoxy ou ribonucléotidiques et fermés soit par une liaison faisant intervenir soit l'extrémité 3', soit l'extrémité 5' de la molécule (figures 1A à 1C et figure 4B). Dans ces molécules en lasso, la partie linéaire peut ne comporter qu'un seul résidu nucléotidique, ou bien peut comporter une chaîne latérale nucléotidique de plusieurs résidus. Dans ces structures, l'un des nucléotides terminaux de l'oligonucléotide est couplé à un nucléotide interne, par une liaison pouvant s'effectuer avec la base ou avec le sucre, ou avec le groupement phosphodiester. De tels oligonucléotides présentent une extrémité libre et une extrémité bloquée. La résistance aux exonucléase sera donc partielle, l'extrémité libre pouvant faire l'objet d'une dégradation nucléolytique. Les extrémités 5' et 3' des oligonucléotides ne sont pas sensibles de façon équivalentes à la dégradation, l'extrémité 3' étant la plus sensible. Un oligonucléotide circulaire en lasso, ne présentant que l'extrémité 5' libre est protégé en grande partie contre la dégradation. De plus, les exonucléase pouvant dégrader la partie linéaire du lasso seront arrêtées au niveau du point de branchement. A ce stade, l'oligonucléotide est devenu totalement résistant aux exonucléases 3' et 5', quelle que soit l'extrémité libre ou branchée initialement. De telles molécules en lasso peuvent comporter des résidus nucléotidiques naturels ou modifiés comme énoncé plus haut au paragraphe précédent. La structure générale des molécules en lasso est présentée en figure 1A et figure 4B.

Font également partie de l'invention des oligonuciéotides fermés en ballon, comme décrits dans la figure 1A, la figure 1B et la figure 4C. Ces oligonucléotides sont fermés par une liaison chimique correspondant à un pontage intercaténaire réalisés entre deux nucléotides internes. Ce pontage peut être effectué par le biais d'un nucléotide réactif-photoactivable par exemple- ou bien en utilisant un réactifs exogène établissant un lien entre deux régions appariées de l'oligonucléotide. Un tel oligonucléotide en ballon ne présente qu'un nombre limité, ou pas, de sites substrats pour des exonucléases. Même si le ou les pontages fermant la molécule oligonucléotidique sont pas effectués au niveau des nucléotides terminaux, seuls seront accessibles aux exonucléases les quelques nucléotides localisés de part et d'autre du point de pontage, aux extrémités de la molécule. Les exonucléases pourront cliver les liaisons phosphodiesters reliant les nucléotides des extrémités non couplées, mais seront arrêtées à partir du site de pontage. Les nucléotides engagés dans le pontage sont résistants aux exonucléase, totalement ou partiellement, et protègent ainsi l'oligonucléotide contre la poursuite de la dégradation nucléolytique.

Font également partie de l'invention une autre famille d'oligonucléotides circulaires refermés sur eux-mêmes par l'intermédiaire d'une molécule non nucléotidique. Ces oligonucléotides fermés possèdent une partie de leurs structure moléculaire correspondant à une séquence d'ADN ou d'ARN dans laquelle les bases sont non modifiées, et dont la première unité nucléosidique est reliée à la dernière par une liaison faisant intervenir une structure molécule quelconque. Par exemple, les oligonucléotides antisens peuvent être circularisés au moyen d'un couplage entre les nucléotides terminaux par une structure protéique ou polypeptidique qui seront liés aux unité nucléosidiques terminale par tout type de couplage (figure 4). Font donc également partie de l'invention des oligonucléotides circulaires comportant une partie nucléotidique et une partie protéique. L'insertion de cette partie protéique peut s'effectuer par divers procédés de couplage. La fraction protéique peut être conçue pour accroître l'efficacité de la molécule nucléotidique par différents mécanismes. La partie protéique pourra, par exemple, favoriser l'internalisation de l'oligonucléotide dans des cellules, permettre de cibler éventuellement certaines cellules en choisissant un déterminant protéique utilisé. Les composants protéiques ou peptidiques utilisés dans les oligonucléotides circulaires de ce type pourront également être des molécules de signalisation, permettant un ciblage intra-cellulaire des oligonucléotides. Par exemple, on pourra utiliser des peptides d'adressage nucléaire provenant de protéines naturelles cellulaires ou virales. Cet aspect de l'invention consiste donc à proposer des oligonucléotides, de structure nouvelle, et comportant des composés susceptibles de réagir spécifiquement avec des récepteurs particuliers de la surface membranaire de la cellule, d'être internalisées par les dites cellules, et d'exercer leur activité biologique à l'intérieur de la cellule.

Font également partie de l'invention des oligonucléotides circulaires refermés sur eux-mêmes par des groupement lipophiles ou des chaînes comportant des radicaux lipophiles et favorisant l'internalisation cellulaire de ces molécules.

Font également partie de l'invention des oligonucléotides fermés associés par des couplages covalents ou non covalents à des structures d'encapsidation de type liposomiques ou de tout autre structure lipoprotéique ou lipopolysaccharidique.

Font également partie de l'invention des oligonucléotides circulaires refermés sur eux-mêmes par une liaison phosphodiester, mais comportant de plus une ou plusieurs liaisons internes produites par des agents réactifs appartenant à la structure de la molécules elle-même ou apportés de façon exogène.

Ces molécules posséderont les caractéristiques de résistance aux exonucléases des oligonucléotides circulaires et offrent de plus des conformations secondaires particulières, pouvant être adaptées à la reconnaissance de sites particuliers sur des cibles présentant elles-mêmes des conformations secondaires ou tertiaires particulières, que ces cibles soient des acides nucléiques ou tout autre structure cellulaire ou virale susceptible de présenter une affinité élective et sélective envers un polynucléotide de structure primaire et secondaire particulière.

Font également partie de l'invention des oligonucléotides antisens circulaires ou fermés susceptibles de former une triple hélice avec l'ARN cible. La formation d'une triple hélice permet de stabiliser l'interaction ADN/ARN entre l'antisens et la séquence cible.
De façon générale, on peut également dire qu'un antisens circulaire est un composé "prodrug" par rapport à un oligonucléotide antisens linéaire. La coupure d'un oligonucléotide circulaire donnera naissance à un oligonucléotide linéaire qui pourra exercer un effet antisens classique, avec retard.

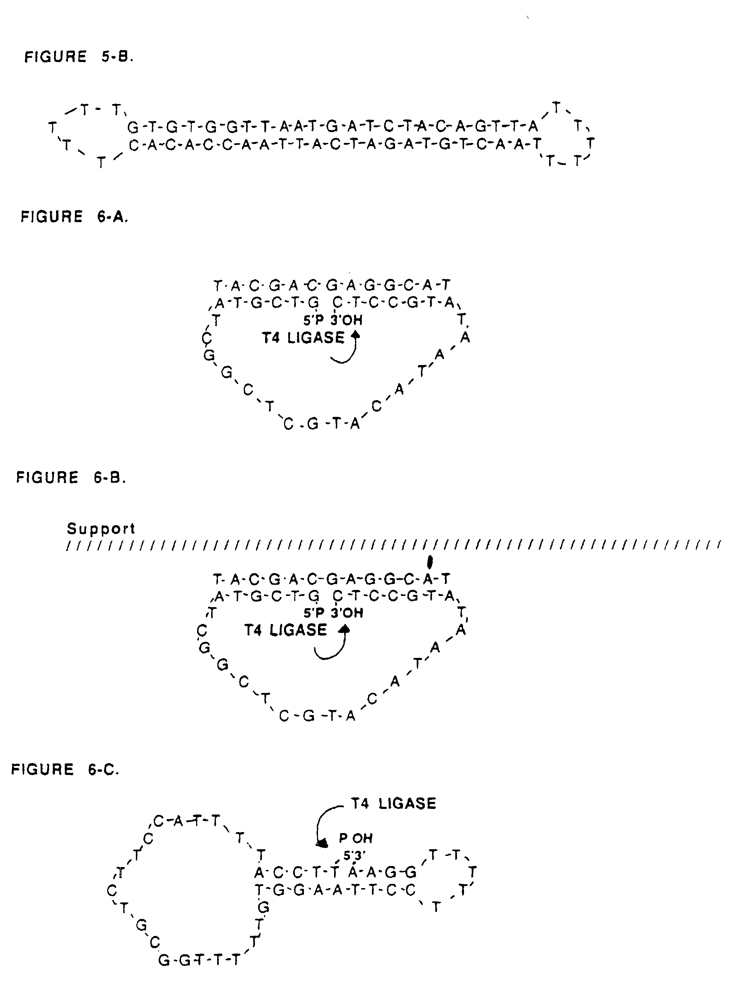

Bien qu'assez souvent les composés selon l'invention comporteront des séquences nucléotidiques non susceptibles de s'auto-apparier font également partie de l'invention des oligonucléotides fermés comportant des régions auto-appariées formant une structure bicaténaire de longueur variable (voir figure 5). Cette structure bicaténaire pourra avoir plusieurs rôles, par exemple celui de stabiliser la molécule pour permettre sa circularisation, lors de la synthèse des molécules circulaires (voir plus loin les procédés de préparations). Cette structure bicaténaire pourra également avoir un rôle actif, comme par exemple celui d'interagir avec des protéines présentant une affinité pour cette séquence. La structure bicaténaire pourrait correspondre à une séquence de fixation pour des facteurs protéiques. En particulier, un aspect de l'invention est de fournir des molécules oligonucléotidiques naturelles stables pouvant fixer des facteurs protéiques cellulaires ou viraux et donc interférer avec des processus biologiques mettant en jeux ces facteurs.
Un exemple de l'utilisation d'oligonucléotides fermés comportant une région auto-appariées -des oligonucléotides destinés à une telle application seront avantageusement circulaires simplement- est la compétition intra-cellulaire avec des facteurs de transcription. Dans ce cas, la partie bicaténaire de l'oligonucléotide fermé portera la séquence de fixation du facteur de transcription, du régulateur, du récepteur nucléaire hormonal que l'on désire piéger. L'interaction entre l'oligonucléotide circulaire bicaténarisé et un facteur de transcription va aboutir à réduire la disponibilité intra-cellulaire de ce facteur, et donc va modifier les équilibres de régulation pour lesquels ce facteur intervient. S'il s'agit d'un facteur de transcription positif, exerçant un effet de stimulation, le blocage de ce facteur aboutira à une inhibition des gènes considérés; s'il s'agit d'un facteur négatif, inhibant l'expression de gènes après interaction avec l'ADN cellulaire, alors l'expression de ces gènes sera stimulée. De façon générale, les oligonucléotides fermés, de type circulaire, de série désoxyribo- et comportant une partie bicaténaire pourront interagir dans des buts thérapeutiques avec tout facteur protéique présentant de l'affinité pour l'ADN et dont on désire réduire ou altérer les effets dans une situation pathologique donnée.
Il est également envisager d'utiliser des oligonucléotides fermés, avantageusement circulaires, pour combiner des approches antisens et sens dans un but de plus grande efficacité. Ce procédé consiste à utiliser simultanément un oligonucléotide antisens fermé dirigé contre l'ARN messager d'un facteur de transcription, et un oligonucléotide "sens" fermé comportant une structure bicaténaire de séquence correspondant au site de fixation sur l'ADN de ce facteur de transcription. Deux niveaux d'action sont donc ciblés simultanément, en synergie, sur la même molécule cible, l'antisens diminuant la synthèse de ce facteur et la molécule sens exerçant un effet de piège sur les molécules résiduelles pouvant subsister. Les approches combinées sens et antisens pourront être utilisées soit pour cibler la même protéine, à deux niveaux d'expression et de fonctionnalité différents, soit pour attaquer deux protéines différentes, dans un but de recherche de la plus grande efficacité du procédé.

Cette approche double peut faire appel à l'utilisation simultanée de deux molécules différentes, l'une sens et l'autre antisens, ou bien à une seule molécule oligonucléotidique fermée comme celle décrite dans la figure 1C.

Un autre exemple de l'utilisation d'oligonucléotides fermés est la compétition intra-cellulaire avec des facteurs d'affinité pour certaines séquences d'ARN bicaténaire. C'est le cas pour certaines protéines de régulation interagissant avec les ARN messagers, en particulier avec les ARN messagers viraux. On peut citer par exemple le cas du produit du gène tat de HIV qui se fixe sur une région bicaténaire le l'extrémité 5' de l'ARN messager viral. Pour être utilisés comme agents interagissant avec tat, dans le cas de cet exemple, les oligonucléotides fermés -circulaires par exemple- comprendraient une partie bicaténaire en série ribo-, le reste de l'oligonucléotide étant composé de nucléotides soit en série ribo- soit en série désoxyribo-.
De façon générale, les oligonucléotides fermés faisant l'objet de l'invention pourront être utilisés comme molécules stabilisées pour le piégeage de facteurs protéiques présentant une affinité pour les acides nucléiques, ADN ou ARN, monocaténaire ou bicaténaire, en mimant à la fois la structure primaire (séquence) des sites d'affinité et les structures secondaires éventuelles (structures en épingle à cheveux, structures cruciformes par exemple).
De tels oligonucléotides fermés, avantageusement circulaires, comportant une séquence nucléotidique reconnue par des facteurs protéiques pourront également porter des groupements réactifs capables d'effectuer des pontages, spontanés ou photo-activables, avec les protéines interagissant avec eux.

Un aspect de l'invention, comme décrit de façon détaillée au paragraphe précédent, est donc de fournir de nouveaux oligonucléotides naturels stables capables d'être internalisés dans les cellules et d'interagir ensuite avec des facteurs cellulaires ou viraux présentant une affinité pour des séquences spécifiques d'acides nucléiques.

Font également partie de l'invention des oligonucléotides circulaires associés deux à deux grâce aux complémentarités de leurs séquences, pour former une structure circulaire bicaténaire complète ou partielle (pouvant présenter des non-appariements, autrement dit des "mismatches"), et résistante aux exonucléases. Ces oligonucléotides bicaténaires circulaires pourront être composé de nucléotides naturels, rares, artificiels, ou modifiés, en série désoxyribo- ou en série ribo-. Ces oligonucléotides circulaires bicaténaires pourraient êtres composés d'ADN de forme I ou d'ADN de forme V, c'est-à-dire comportant des structures composites de type B et Z. L'ADN de forme I est de l'ADN circulaire super-enroulé, dont les deux brins présentent un entrelacement. L'ADN de forme V est un ADN dont les brins complémentaires ne sont pas entrelacés, c'est-à-dire un ADN formé de deux brins complémentaires, côte-à-côte. L'ADN bicaténaire peut adopter des conformations différentes, A, B ou Z. La forme la plus naturelle et la plus courante de l'ADN est une hélice droite de type B, alors que la forme Z, moins fréquente, est une hélice gauche, plus allongée que la forme B. Une structure circulaire telle que celle décrite ci-dessus, comportant deux brins complémentaires non entrelacés comportera à la fois des hélices gauches et des hélices droites. Un tel oligonucléotide circulaire bicaténaire pourra être utilisé comme agent de type sens, pour interagir avec des protéines présentant de l'affinité pour une séquence particulière qui sera présente sur l'oligonucléotide considéré. Un tel oligonucléotide bicaténaire circulaire, en série ribo en particulier, pourra également être utilisé comme immunostimulant de façon générale, et plus particulièrement comme agent d'induction d'interférons. A noter que cette fonction d'immunostimulation potentielle que présente certains ARNs pourra également être exploitée en utilisant des oligonucléotides circulaires monocaténaires prés entant des structures bicaténaires auto-appariées grâce à des complémentarités internes de séquence et en tirant parti de l'avantage que confère la résistance aux exonucléases d'une structure circulaire.

### III. PRÉPARATION DES OLIGONUCLÉOTIDES FERMÉS

Les oligonucléotides fermés qui font l'objet de l'invention peuvent être obtenus par voie chimique, biologique, ou par des approches faisant appel à des combinaisons des techniques de la chimie de synthèse et de la biologie moléculaire.

Les oligonucléotides fermés peuvent donc être préparés, soit à partir d'oligonucléotides linéaires, refermés ensuite par des techniques chimiques ou biologiques, soit directement en tant qu'oligonucléotides par voie chimique, utilisant des réaction aboutissant à la cyclisation de la molécule. Ces deux approches sont considérées successivement ci-dessous.

### 1-PRÉPARATION D'OLIGONUCLÉOTIDES CIRCULAIRES A PARTIR D'OLIGONUCLÉOTIDES LINÉAIRES PAR LES TECHNIQUES DE LIGATION

Diverses méthodes de synthèse chimique d'oligonucléotides naturels ont été développées et sont bien connu des spécialistes travaillant dans les règles de l'art. Par exemple, une méthode consiste à utiliser un support solide dit CPG (controlled pore glass) sur lequel le premier nucléoside est fixé covalemment par un bras de couplage, par l'intermédiaire de son extrémité 3'OH. L'extrémité 5'OH du nucléoside est protégé par un groupe di-p-méthoxytrityl acido-labile. Cette approche, utilisant la chimie des phosphites triesters, et dans laquelle des désoxynucléosides 3'phosphoramidites sont utilisés comme synthons est appelée la méthode des phosphoramidites (Caruthers, 1985). Cette approche est la plus utilisée actuellement et présente l'avantage d'être entièrement automatique. La synthése d'un oligonucléotide à partir d'une première unité fixée sur CPG commence par une étape de déprotection au cours de laquelle le groupe trityl est éliminé, puis une unité nucléosidique activée sur son groupe 5' est additionnée, les produits non-réactifs sont bloqués, puis un nouveau cycle de déprotection/activation/couplage recommence. Typiquement, l'addition d'un déoxynucléotide s'effectue selon les quatres étapes suivantes i) déprotection et élimination d'un groupe protecteur diméthoxytrityl par l'acide , ii) condensation du produit résultant avec un désoxynucléoside 3'-phosphoramidite, donnant un désoxydinucléoside phosphite triester, iii) acylation, c'est-à-dire blocage des groupements 5'-hydroxyl n'ayant pas réagit et iv) oxydation du phosphite triester en phosphate triester. La répétition de tels cycles conduit à la synthèse d'oligonucléotides pouvant atteindre plus de 200 unités.
Pour citer un second exemple, une autre approche utilisée pour la synthèse d'oligonucléotides est celle de la chimie des phosphonates (Froehler et al, 1986). Cette approche commence par la condensation d'un désoxynucléoside 3'-H-phosphonate avec un deoxynucléoside couplé à un support de verre de silice. Des cycles de condensation successifs conduisent à la synthèse d'oligonucléotides H-phosphonates. Ces oligonucléotides sont oxydés en une étape pour donner les phosphodiesters.

En utilisant l'une ou l'autre de ces techniques, ou tout autre procédure séquentielle permettant la synthèse chimique de chaînes polynucléotidiques de séquence déterminée à l'avance, on obtient des oligonucléotides linéaires qu'il est possible de circulariser par voie biologique, en utilisant des enzymes de ligation. Pour pouvoir utiliser des ligases pour refermer les oligonucléotides sur eux-mêmes, les oligonucléotides doivent comporter un groupement terminal 5'P, que la phosphorylation du 5' terminal ait été effectué par voie chimique, ou bien par voie biologique en utilisant une kinase (préférentiellement la polynucléotide kinase) et de l'ATP ou tout autre donneur de phosphate.

Différentes procédures convenant pour effectuer avantageusement la circularisation d'oligonucléotides linéaires sont décrites ci-dessous

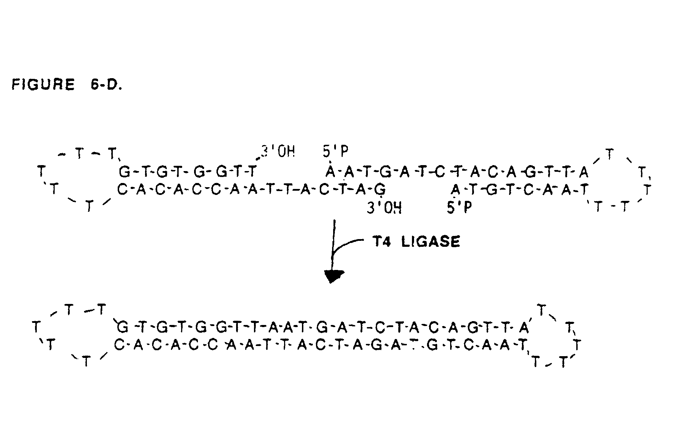

1-1-Un oligonucléotide linéaire peut être circularisé en établissant une structure partiellement bicaténaire appariée pour permettre le fonctionnement d'une ligase, comme la T4 ligase, au moyen d'un second oligonucléotide plus court que le précédent, et de séquence complémentaires aux deux extrémités du premier oligonucléotide. Dans ce cas, illustré figure 6, le second petit oligonucléotide fait office d'adaptateur et permet la mise bout à bout des deux nucléotides terminaux de l'oligonucléotide à circulariser. L'action de la T4 ligase, ou de tout autre enzyme de ligation capable d'exercer son action sur l'ADN permet alors la circularisation en formant une liaison phosphodiester entre ces deux nucléotides. Cette ligation peut se faire en solution, les deux oligonucléotides étant mélangés dans un milieu permettant l'hybridation et la ligation dans des conditions de concentration et de température convenable pour favoriser la circularisation intra-caténaire et défavoriser les ligation inter-oligonucléotidiques pouvant diminuer le rendement de la réaction de circularisation proprement dite.

1-2-Une variante du procédé décrit au (1) consiste a effectuer la circularisation de l'oligonucléotide toujours grâce à un adaptateur, mais en utilisant un oligonucléotide fixé sur un support pouvant être par exemple de la nitrocellulose ou un dérivé, une membrane de nylon, un support de verre, une structure polysaccharidique, ou tout autre support permettant de fixer de façon covalente ou non covalente un fragment d'acide nucléique et permettant ultérieurement l'hybridation entre ce fragment et un oligonucléotide et compatible avec l'action d'une ligase. Cette procédure consiste donc à fixer l'oligonucléotide adaptateur sur un support, soit au moyen d'une liaison covalente, soit par des liens non-covalents. On utilisera de manière avantageuse les liaisons covalentes permettant d'effectuer un grand nombre de cycles d'hybridation/ligation avec l'oligonucléotide à circulariser. L'oligonucléotide adaptateur pourra être fixé à son support soit au niveau d'un nucléotide terminal, directement ou au moyen d'un agent de couplage, soit par un nucléotide situé en position interne et portant un groupement réactif. Le schéma de principe d'une telle méthode est illustré figure 6. L'intérêt de fixer l'oligonucléotide adaptateur est double: d'une part, cette fixation effectuée dans des conditions physiques contrôlées (nombre de molécules fixées par unité de surface) permet de réduire l'incidence de la formation de concatémères lors de la réaction d'hybridation, à la suite de liaison inter-oligonucléotidiques et, d'autre part, elle facilite la réalisation de réacteurs de ligation utilisant un grand nombre de fois les oligonucléotides adaptateurs pour de multiples cycles de circularisation.

1-3-Les oligonucléotides pourront être circularisés en tirant parti de structures secondaires délibérément introduites en prévoyant des séquences capables de se replier sur elles-mêmes en formant des structures bicaténaires partielles. Par exemple, la figure 6 illustre le cas d'un oligonucléotide "en haltère", comportant une partie linéaire formant une boucle et comportant la séquence de type antisens, une région bicaténaire longue de 9 paires de bases, et une séquence de fermeture composée de T₅. Cette structure est capable d'effectuer des auto-appariements donnant ainsi une molécule susceptible d'être utilisée comme substrat par un enzyme de ligation comme la T4 ligase. Dans ce cas, le rendement de la ligation dépend entre autres de la stabilité de l'appariement au niveau de la structure bicaténaire. Plusieurs séquences d'appariement ont fait l'objet d'études comparatives et l'une des séquences permettant un rendement de circularisation avantageux (de l'ordre de 75%) est indiquée dans la figure 6. Bien que n'importe lesquels des nucléotides puissent êtres utilisés pour réaliser une boucle de fermeture de longueur variable, on utilisera préférentiellement de 4 à 8 résidus, et principalement soit A soit T.
La région bicaténaire d'appariement pourra comporter soit une séquence utilisée seulement pour la formation de la structure en "haltère", soit une séquence correspondant en partie à la région cible et potentiellement déplaçable par une hybridation inter-moléculaire.
Les conditions expérimentales permettant la circularisation efficace de l'oligonucléotide dont la séquence est donnée en figure 6 sont indiquées précisément plus loin dans la partie expérimentale (voir "Propriétés et Avantages des Oligonucléotides Fermés). Dans le cas de cette séquence, le rendement de circularisation est de l'ordre de 75%.
Cette technique a été utilisée pour préparer les oligonucléotides circulaires dont il est question dans la partie expérimentale

1-4-Les oligonucléotides circulaires pourront également être formés par une structure bicaténaires refermée sur elle-même à chaque extrémité par une courte boucle de nucléotides de jonction. Ces oligonucléotides pourront être utilisés pour des approches de type "sens" telles celles décrites plus haut. Un exemple typique d'un oligonucléotide sens est illustré figure 6. Cet oligonucléotide comporte une séquence appariée de 24 nucléotides et deux boucles de jonction formées par T₅. Dans l'exemple donné ici, cet oligonucléotide circulaire comporte une structure bicaténaire correspondant à la séquence de reconnaissance du facteur de transcription hépatocytaire HNF-1. Un tel oligonucléotide peut être circularisé simplement en tirant partie de la structure secondaire bicaténaire formée par les séquence complémentaires. Le point de fermeture (c'est-à-dire les extrémités) de l'oligonucléotide seront choisies de façon a permettre la meilleure efficacité de circularisation par repliement intra-moléculaire. Ce point pourra être centré ou plus ou moins distal par rapport à la médiane de la structure secondaire centrale. Il convient également de noter que de tels oligonucléotides peuvent être synthétisé à partir non pas d'une réaction intra-moléculaire, mais par une réaction intermoléculaire, en utilisant deux oligonucléotides linéaires repliés sur eux-mêmes, présentant une structure bicaténaire partielle et générant des bouts cohésif, pouvant s'apparier entre-eux (voir le schéma en figure 6).

1-5-Pourra être utilisée pour cycliser des oligonucléotides devant comporter une région centrale bicaténaire une technique consistant à préparer deux oligonucléotides complémentaires, l'un long et l'autre court, le second hybridant dans la partie centrale du premier. Par l'action de la ligase de T4, il est possible de joindre les deux extrémités du long oligonucléotides aux extrémités de l'oligonucléotide apparié, même si il n'y a pas d'homologies de séquence permettant la formation d'un auto-appariement au niveau des séquences distales 3' et 5'. Un tel mécanisme aboutit à la formation d'une structure oligonucléotidique circulaire fermée comportant une partie bicaténaire centrale.

1-6-Pour la préparation d'oligonucléotides circulaires en série ribo- (oligoribonucléotides) à partir d'oligoribonucléotides linéaires, les techniques utilisables sont de même ordre que celles décrites ci-dessus. Cependant il est également possible d'utiliser un enzyme, la RNA ligase de T4 qui est capable d'effectuer spontanément des circularisation d'oligonucléotides en série ribo- ou désoxyribo-. Cet enzyme permet, en présence d'ATP, de clore des oligonucléotides linéaires et de les convertir en oligonucléotides circulaires. Il est donc possible, sans matrice particulière, et en l'absence de tout oligonucléotide adaptateur de circulariser ainsi des oligoribonucléotides. Ce même enzyme permet également de circulariser des oligodésoxyribonucléotides, mais avec une efficacité beaucoup moins élevée que lorsqu'il s'agit d'oligoribonucléotides. La RNA T4 ligase sera employée avantageusement pour circulariser des oligonucléotides antisens comportant une activité ribozymique, ou bien pour circulariser des ARN "sens", comme par exemple les séquences d'interaction avec des transactivateurs de type tat de HIV.

1-7-Les procédures décrites ci-dessus font toutes intervenir des enzymes de ligation pour former des liaisons phosphodiesters et clore les molécules linéaires. Pourront être utilisées pour circulariser des oligonucléotides dans le but de les rendre résistant à l'action des nucléases, dans l'esprit de l'invention décrite ici, tout enzyme permettant la formation d'une liaison phosphodiester entre deux résidus nucléotidiques, qu'il s'agisse de DNA ligases ou de RNA ligases. En particulier, pourront être utilisées avantageusement des enzymes thermostables, provenant d'organismes thermorésistants, permettant d'effectuer des cycles successifs de ligation/dénaturation/hybridation. Pourront être utilisées pour préparer les molécules circulaires faisant l'objet de l'invention, soit des enzymes de ligation en solution, soit des enzymes fixées sur un support dans le but d'augmenter le rendement de la réaction et/ou d'en diminuer le coût.
Pourront également être utilisées pour préparer les molécules circulaires faisant l'objet de l'invention tout réactif chimique permettant une ligation chimique. Par exemple, et de façon non limitative, pourraient être utilisés des réactifs tels que le carbodiimide ou le bromure de cyanogène.

### 2 AUTRES VOIES CHIMIQUE DE PRÉPARATION DES OLIGONUCLÉOTIDES CIRCULAIRES ET/OU FERMÉS

La préparation d'oligonucléotides fermés dans un but d'application pharmacologiques, que ce soit pour des expérimentations sur l'animal ou pour la préparation de composés pharmaceutiques utilisera avantageusement des méthodes chimiques permettant la préparation des quantités importantes requises.
Plusieurs voies pourront êtres utilisées, en particulier on pourra soit synthétiser des oligonucléotides linéaires par les voies usuelles que l'on fermera ensuite par ligation chimique ou par une liaison faisant intervenir des nucléotides terminaux, soit synthétiser des oligonucléotides pouvant faire l'objet d'une cyclisation directement en dernière étape de la synthèse chimique.

### 2-1-Synthèse chimique d'oligonucléotides cycliques

Plusieurs procédures de préparation d'oligonucléotides cycliques ont été décrites (Barbato et al, 1989, de Vroom et al., 1988). Ces approches, en phase liquide ou sur support, permettent d'obtenir de courts oligonucléotides cycliques. Par exemple, on peut utiliser une technique consistant à fixer un premier résidu nucléotidique par l'intermédiaire du groupe amine exocyclique. A partir d'un tel support, l'assemblage de l'oligonucléotide peut s'effectuer à partir des deux extrémités 3' et 5' qui sont protégés et disponibles. La cyclisation est effectuée, un fois la synthèse achevée, en utilisant par exemple du MSNT, après déblocage des groupements protecteurs des deux extrémités.

Pourront être utilisées pour synthétiser des oligonucléotides cycliques dans le but de fabriquer des molécules antisens ou sens résistantes aux exonucléases, tout procédé de synthèse permettant l'élongation d'une chaîne polynucléotidique à partir des deux extrémités 3' et 5', et la réunion de ces deux extrémités après achèvement de l'élongation de la séquence choisie. Pourront être utilisés tout procédés permettant la réunion chimique de deux oligonucléotides linéaires synthétisés indépendamment et réassociés ensuite pour former une structure fermée au moyen d'une réaction chimique spécifique des extrémités.

### 2-2-Fermeture et cyclisation d'oligonucléotides linéaire

Divers procédés pourront être utilisés pour fermer des oligonucléotides linéaires afin de former des structures closes faisant l'objet de l'invention. Dans ces structures, l'un, l'autre, ou les deux nucléotides terminaux seront engagées dans une liaison de couplage. En dehors des structures strictement circulaires décrites plus haut, et que l'on pourra obtenir soit par ligation soit par tout autre réaction chimique, diverses d'autres structures fermées déjà évoquées pourront êtres obtenues par couplage chimique.
C'est le cas en particulier des structures en lasso ou l'un des nucléotides terminaux -avantageusement le nucléotide 3' terminal- est couplé à l'un des nucléotides de la partie 5' de l'oligonucléotide. De telles structures pourront être obtenues en utilisant des nucléotides modifiés capables d'établir des pontages avec d'autres parties de la molécule. C'est le cas également des structures en ballon, dans lesquelles un pontage a été établit par tout agent entre deux ou plus de deux nucléotides situés dans les régions terminales de l'oligonucléotide, ces région étant appariés un petit nombre de nucléotides, préférentiellement sur 4 à 10 nucléotides.
C'est le cas également des oligonucléotides qui seront fermés par l'intermédiaire de tout groupement, toute molécule ou macromolécule permettant un couplage des nucléotides terminaux entre-eux, ou entre un nucléotide terminal et un nucléotide interne, et pouvant accroître l'efficacité du composé en terme de son action intra-cellulaire antisens ou sens. A titre d'exemple, on pourra utiliser pour cycliser les oligonucléotide des polypeptides longs par exemple de 5 à 100 acides aminés, l'enchaînement polypeptidique devant avoir une masse suffisante pour être reconnu par des récepteurs cellulaires et permettre une meilleure internalisation ou un meilleur adressage.

### IV. EXEMPLES D'APPLICATION

Les oligonucléotides fermés, en particulier circulaires, faisant l'objet de l'invention décrite ici pourront êtres utilisés dans tous les cas ou il sera avantageux de disposer d'un oligonucléotide présentant une résistance accrue aux exonucléases par rapport à un oligonucléotide linéaire.

Les oligonucléotides fermés, pourront en particulier être utilisés comme agents antisens ou sens pour agir de façon spécifique sur la transcription ou la traduction de protéine(s) dont on désire moduler le niveau d'expression dans un but de recherche ou de thérapeutique.

Les quelques applications possibles qui sont donnés ci-dessous ne sont que des exemples non-exhaustifs et en aucun cas limitatifs de situations dans lesquelles une approche de type antisens, ou sens, pourrait être envisageable, et où l'utilisation d'oligonucléotides naturels résistants aux exonucléases et présentant une toxicité des plus réduites offrirait un avantage.

Plus particulièrement, les composés selon l'invention sont utilisables à titre d'agent thérapeutique, notamment comme agent anti-viral ou anti-cancéreux, en particulier dans des compositions pharmaceutiques pour usage topique externe, ou pour usage systémique.

Mais ils peuvent également être des inducteurs d'immunomodulateurs naturels tels que l'interféron. Il est possible également de les utiliser en diagnostic in vitro ou in vivo

De façon générale, les oligonucléotides fermés, naturels et circulaires en particuliers, trouveront des terrains d'applications particulièrement adaptés dans le domaine de la dermatologie en raison de l'accessibilité des cibles à traiter, et de la toxicité mineure ou inexistante de ces composés. Toutes les affections dermatologiques, pouvant relever d'un mécanisme de dysfonctionnement génétique pour lesquelles on pourra identifier un facteur étiologique, en connaître la séquence du gène, et/ou de l'ARN messager permettra d'envisager potentiellement une approche antisens, -ou même une approche "sens" dans certains cas favorables-.
L'utilisation d'oligonucléotides naturels, donc présentant la toxicité la plus réduite, permet d'envisager la possibilité de ce genre d'approche pour des affections graves ou mineures, et même éventuellement pour des utilisations de type cosmétologiques, c'est-à-dire sur peau saine, et dans un domaine d'application ou les toxicités des produits doivent êtres les plus basses possibles.

A côté des cibles virales définies plus loin, de nombreuses maladies dermatologiques pourraient êtres traitées par des oligonucléotides naturels circulaires ou fermés résistants aux exonucléases. Parmi les cibles potentielles de telles approches, on peut noter les maladies inflammatoires telles que les dermatites atopiques ou le lupus érythémateux, les maladies de la kératinisation comme l'ichtyose et le psoriasis, et les maladies tumorales comme le mélanome ou le lymphome T cutané. Ainsi par exemple, des oligonucléotides antisens circulaires appliqués à la dermatologie pourraient être dirigés contre des ARN messagers de médiateurs de l'inflammation comme des interleukines, contre des RNA messagers de protéines impliquées dans les troubles de la prolifération des cellules épidermiques, ou bien contre des ARN messagers codant pour des protéines impliqués éventuellement dans le vieillissement cutané phénotypique, comme par exemple la collagénase, l'élastase, les transglutaminases.

De façon plus générale, les oligonucléotides fermés, naturels et circulaires principalement, pourraient par exemple être utilisés comme agents antiviraux, antisens, ou sens, que ce soit pour des indications topiques (dermatologiques) ou pour des indications systémiques. Par exemple, de tels oligonucléotides pourraient êtres utilisés comme agents anti-herpétiques (HSV 1 et HSV 2, CMV, EBV), comme agents anti-papillomavirus (HPV cutanés, génitaux, laryngés ou autres), comme agents anti-hépatites (HBV, HCV, HDV), comme agents anti-HIV (HIV-1 et HIV-2), comme agent anti-HTLV (HTLV-1 ou HTLV-2), etc..
Ces oligonucléotides circulaires, pourraient également être utilisés comme agents de répression de l'expression de certaines protéines cellulaires directement responsables ou impliquées dans l'étiologie de maladies de la prolifération et de la différenciation cellulaire. Par exemple, ces oligonucléotides circulaires pourraient être dirigés contre l'expression d'oncogènes cellulaires hyper-exprimés ou exprimés de façon incontrôlés dans des types cellulaires tumoraux (RAS, ERB, NEU, SIS, MYC, MYB,etc...).
En particulier, ces oligonucléotides circulaires naturels résistants aux exonucléases sériques pourraient êtres utilisés comme agent antisens dirigés contre des ARN messagers d'oncogènes exprimés dans des cellules leucémiques et impliqués dans leur prolifération, ou bien comme agents "sens" dirigés contre des protéines à affinité pour certaines séquences de l'ADN et exprimées à des niveaux pathologiques dans certaines de ces maladies prolifératives. Dans le cadre du traitement de certaines leucémies, pour des greffes de moelle, les oligonucléotides fermés, circulaires, pourront êtres utilisés dans le cadre d'applications "ex vivo".

Pour ces nombreuses indications, des formulations galéniques adéquates pourront êtres établies afin d'optimiser la délivrance de ces molécules à leurs cellules cibles. Ainsi, par exemple, des oligonucléotides fermés, circulaires en particulier, pourront êtres encapsulés dans des liposomes, des nano-particules, des particules LDL, ou dans tout autre type de micro-sphère permettant une conservation adéquate, et favorisant le ciblage. Les molécules oligonucléotidiques fermées, circulaires par exemple, pourraient également être associées à des agents surfactants cationiques. Il est bien évident que ces quelques exemples ne sont ni exhaustifs ni limitatifs.

Les oligonucléotides fermés, circulaires en particulier, faisant l'objet de l'invention décrite ici, sont donc susceptibles d'êtres inclus dans toutes sortes de préparations pharmaceutiques, à des concentrations variables selon les indications.
En particulier, les applications dermatologiques évoquées plus haut exigeront la préparation de crèmes, solutions, émulsions, lotions, gels, sprays, poudres, aérosols, etc.., préparés en associant l'oligonucléotide circulaire -ou fermé- de séquence choisie, avec les composants pharmaceutiques usuels entrants dans la composition de ces produits. Par exemple, pour des préparations destinées à des applications topiques en dermatologie, les oligonucléotides circulaires, ou fermés, pourront être associés à toutes sortes d'agents de préservation, comme l'hydroxybenzoate de méthyle ou de propyle, le chlorure de benzalkonium par exemple, et à d'autres agents de stabilisation, d'émulsification, de dispersion, de suspension, de solubilisation, de coloration, d'épaississement, de fragrances, etc...
On doit noter que certaines de ces compositions, en particulier les compositions destinées à des applications topiques pour des indications en dermatologie, pourront associer à la fois des oligonucléotides circulaires -ou fermés- avec d'autres principes actifs comme par exemple des agents bactériostatiques, ou antiseptiques, ou antimicrobiens, ou antibiotiques, ou analgésiques, ou antipruritiques, etc...
Tous ces exemples ne sont donnés que pour illustrer le propos et ne sont ni exhaustifs ni limitatifs.

### V. PROPRIETES ET AVANTAGES DES OLIGONUCLEOTIDES FERMES

Les exemples expérimentaux sont donnés ci-dessous pour illustrer les avantages d'un oligonucléotide fermé sur un oligonucléotide "ouvert", linéaire, de même séquence. Ces exemples sont tirés d'expériences réalisées avec des oligonucléotides circularisés selon le procédé indiqué dans la partie "obtention des oligonucléotides fermés", paragraphe 1-3. Les oligonucléotides fermés dont il est question ici sont donc des oligonucléotides circulaires, composés de nucléotides naturels, reliés entre eux par des liaisons phosphodiester non modifiées.

La description et les exemples font référence aux figures ci-annexées sur lesquelles portent les légendes suivantes :

### Figure 1:

A-Exemples de structures d'oligonucléotides antisens fermés. B-Exemples de structures d'oligonucléotides sens fermés.

C-Molécule mixte pouvant exercer un effet sens et antisens.

D-Structure des bases composant les oligonucléotides et structure de la liaison phosphodiester reliant les nucléotides naturels entre eux.

### Figure 2:

A-Représentation schématique de certaines des modifications pouvant être utilisées pour accroître la stabilité de la liaison phosphodiester et en particulier sa résistance aux endonucléases.

B-Représentation de quelques agents réactifs de type intercalants pouvant êtres couplés aux oligonucléotides antisens.

### Figure 3:

Exemple d'un ribozyme circulaire comportant le site catalytique dit "en T" et une boucle d'ARN pouvant être complémentaire à une séquence cible.

### Figure 4:

A-Représentation schématique d'un oligonucléotide circulaire composé de nucléotides naturels reliés entre-eux par des liaisons phosphodiesters non modifiées.

B-Représentation schématique d'un nucléotide fermé en lasso avec extrémité 5' libre et extrémité 3' bloquée dans le pontage intra-caténaire. C-Représentation schématique d'un oligonucléotide fermé en ballon, avec une liaison intra-caténaire établie entre les pénultièmes nucléotides terminaux 5' et 3'. Ce type de structure peut comporter une ou plusieurs liaisons intercaténaires entre l'une quelconque des paires de nucléotides appariés formant la queue du ballon.

D-Représentation schématique d'un oligonucléotide fermé par un peptide (oligopeptide).

E-Représentation schématique d'un oligonucléotide circulaire comportant une extension peptidique latérale.

### Figure 5:

A-Représentation schématique d'un oligonucléotide fermé, circulaire, comportant une région bicaténaire auto-appariée partielle.

B-Représentation schématique d'un oligonucléotide fermé, circulaire, composé d'une longue séquence bicaténaire auto-appariée (séquence de reconnaissance d'un facteur de transcription par exemple) et de deux boucles de jonction poly(T).

### Figure 6-

A-Circularisation d'un oligonucléotide linéaire en utilisant un oligonucléotide partiellement complémentaire comme guide et la ligase de T4.

B-Circularisation d'un oligonucléotide linéaire en utilisant un oligonucléotide partiellement complémentaire comme guide fixé sur un support et la ligase de T4.

C-Circularisation d'un oligonucléotide comportant une région d'auto-appariement au moyen de la ligase de T4.

D-Exemple de réaction bimoléculaire entre deux oligonucléotides auto-appariés et présentant des bouts cohésifs, permettant de générer un oligonucléotide portant une longue région bicaténaire et deux boucles d'adaptation.

### Figure 7:

A-Autoradiographie d'un gel de polyacrylamide sur lequel on a fait migrer divers oligonucléotides (circulaires et linéaires) après traitement par différents enzymes exonucléolytiques.
La séquence de l'oligonucléotide c7 est celle décrite dans la Figure 6C; les oligonucléotides c6 et c8 sont composés de séquences différentes mais pouvant adopter le même type de structure que c7, c'est-à-dire une structure fermée avec partie bicaténaire centrale.
Les conditions de ligation des oligonucléotides c6, c7 et c8 sont celles décrites dans "Propriétés et avantages des oligonucléotides fermés -1". 1 µg d'oligonucléotide radioactif provenant de la réaction de ligation (pistes 5 à 8) ou 1 µg d'oligonucléotide linéaire correspondant (pistes 1 à 4) ont été analysés sur gel de polyacrylamide 15%/7M urée sans traitement postérieur (pistes 4 et 5), ou après incubation avec la phosphatase alcaline (pistes 3 et 6), avec l'exonucléase VII (pistes 2 et 7) ou avec la phosphodiésterase I (pistes 1 et 8) dans les conditions décrites dans "Propriétés et avantages des oligonucléotides fermés -2".
L indique la position de migration des oligonucléotides linéaires ; F indique la position de migration des oligonucléotides fermés.

B-Autoradiographie d'un gel de polyacrylamide sur lequel on a fait migrer des oligonucléotides linéaires ou circulaires après incubation en présence de sérum.
1 µg de l'oligonucléotide radioactif c7 ( dont la séquence est donnée en figure 6C) linéaire (L) ou fermé (F), ont été préparés et purifiés comme décrit dans "Propriétés et avantages des oligonucléotides fermés-1". Après incubation à 37°C pendant les temps indiqués en présence de DMEM contenant 10% sérum de veau foetal, les produits ont été analysés sur gel de polyacrylamide 15%/7M urée dans les conditions détaillées dans "Propriétés et avantages des oligonucléotides fermés-3".

C-Représentation graphique de la dégradation (mesurée par analyse sur gel comme décrit précédemment) des oligonucléotides c7L (linéaires) et c7F (circulaires) après incubation dans 10% de sérum de veau foetal, comme décrit précédemment, du temps t=0 au temps t=96 heures Pour obtenir une quantification, les fragments de gel correspondant aux localisation des bandes, observées par autoradiographie, ont été découpées et leur radioactivité mesurée par comptage dans un compteur à scintillation. On exprime les résultats en pourcentage de dégradation par rapport à la radioactivité du temps t=0.

### Figure 8:

A-Autoradiographie d'un gel de polyacrylamide non dénaturant sur lequel on a fait migrer des oligonucléotides linéaires
ou circulaires après hybridation avec des oligonucléotides comportant des séquences complémentaires, en série désoxyribo- ou ribo-.
300 ng de l'oligonucléotide radioactif c7 fermé (F) ou 160 ng de l'oligonucléotide c7 linéaire (L) ont été incubés avec des quantités croissantes d'un oligonucléotide non complémentaire (pistes a à g) ou d'un oligonucléotide 42-mer (12; pistes h à n) complémentaire de 21 nucléotides de la grande boucle de c7 (Figure 6C). La quantité d'oligonucléotide froid ajoutée est de 0 (pistes a et h), 30 ng (b et i), 60 ng (c et j), 150 ng (d et k), 300 ng (e et 1), 750 ng (f et m) ou 1500 ng (g et n). Après hybridation dans les conditions décrites dans "Propriétés et avantages des oligonucléotides fermés-4-1", les produits ont été analysés sur gel non dénaturant de polyacrylamide 20%.
Les flèches indiquent les positions des oligonucléotides c7 fermé (F), linéaire (L) et des hybrides c7L/12 et c7F/12.

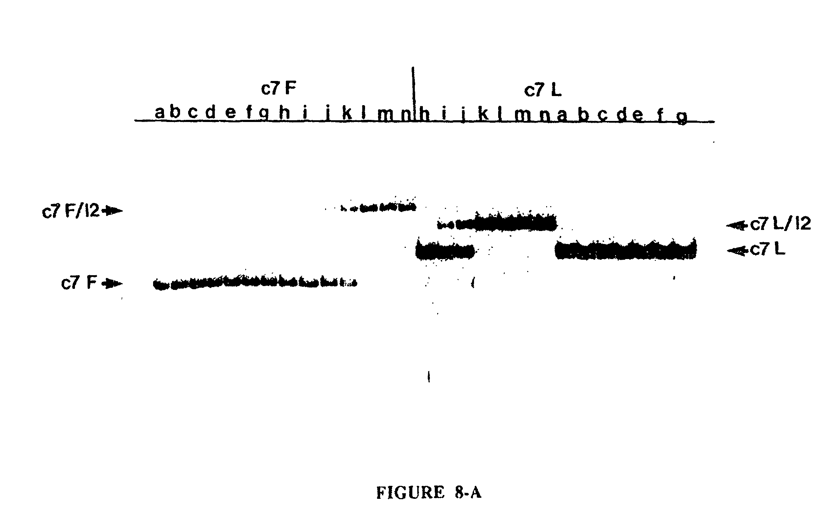

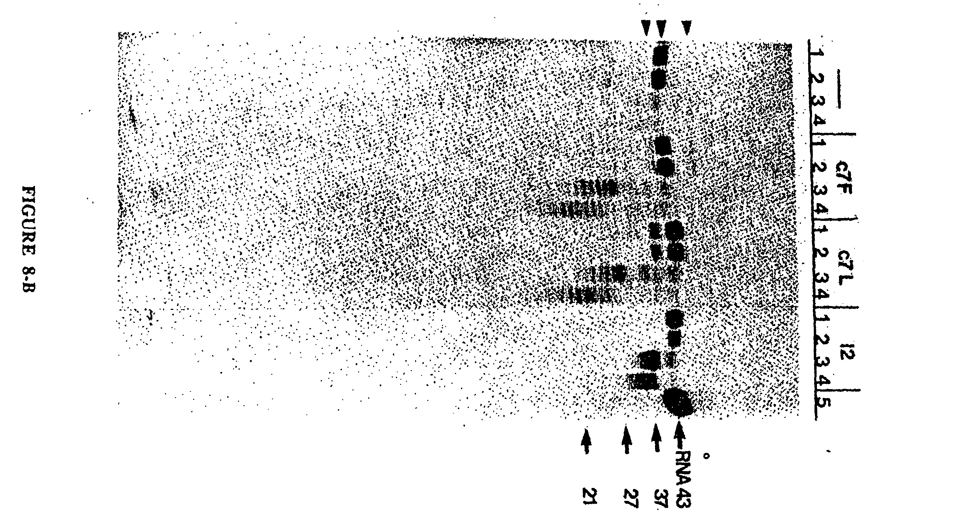

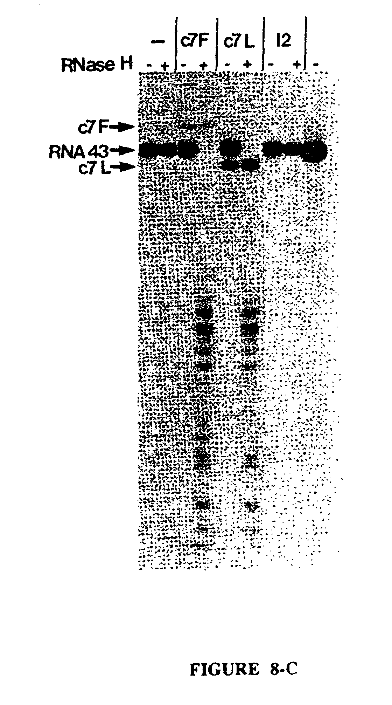

B-Autoradiographie d'un gel de polyacrylamide dénaturant sur lequel on a fait migrer des oligonucléotides linéaires ou circulaires hybridés ou non avec des oligoribonucléotides comportant une région complémentaire puis traités à la nucléase S1.
300 ng de l'oligonucléotide c7 fermé (F) ou linéaire (L), synthétisés et purifiés comme décrit dans la Figure 8A, ou 300 ng d'un oligonucléotide froid 42-mer (12) ont été incubés avec 1 ng d'un ARN long de 43 nucléotides transcrit *in vitro* et marqué au ³²P à haute activité spécifique ("Propriétés et avantages des oligonucléotides fermés-4-2"). L'ARN 43-mer comporte 27 bases complémentaires des 21 nucléotides de la grande boucle plus 6 nucléotides de la région auto-appariée de c7F (Figure 6C); il est aussi complémentaire de 37 bases de l'oligonucléotide 42-mer 12. Après hybridation, les produits sont analysés sur gel de polyacrylamide 20%/7M urée directement (piste 1) ou après incubation de 3 minutes (piste 3) ou 30 minutes (piste 4) en présence de la nucléase S1 ou 30 minutes en absence de nucléase S1 (piste 2) dans les conditions décrites dans "Propriétés et avantages des oligonucléotides fermés-4-2".La partie gauche de la figure (-) montre les résultats obtenus avec l'ARN 43-mer incubé seul.
Piste 5: ARN 43-mer n'ayant subi aucun traitement postérieur à la transcription.
Les flèches indiquent les positions des oligonucléotides ARN 43-mer, c7 fermé (F), c7 linéaire (L) ainsi que celles des ARN protégés (RNA 37, 27 et 21).

C-Autoradiographie d'un gel de polyacrylamide dénaturant sur lequel on a fait migrer des oligonucléotides linéaires ou circulaires hybridés ou non avec des oligoribonucléotides comportant une région complémentaire et que l'on a ensuite traités à la RNase H.
Les oligonucléotides testés (RNA 43, c7F , C7L et 12), ainsi que les conditions d'hybridation, sont les mêmes que ceux décrit dans la Figure 8B. Après hybridation, les produits de la réaction ont été incubés en absence (-) ou présence (+) de la RNase H et ont été analysés sur gel de polyacrylamide 20%/7M urée dans les conditions décrites dans "Propriétés et avantages des oligonucléotides fermés-5". Les flèches indiquent les positions de l'ARN 43-mer, c7 fermé (F) ou c7 linéaire (L).

### Figure 9 :

Analyse des effets d'oligonucléotides linéaires ou circulaires sur la croissance cellulaire.

### Figure 10 :

Etude des effets inhibiteurs d'oligonucléotides antisens linéaires ou circulaires sur la multiplication du virus HSV-1.

### Figure 11 :

Gel de retard illustrant la fixation du facteur de transcription HNF-1 par des oligonucléotides de type sens, linéaires bicaténaires ou fermés circularisation. Les abbréviations utilisées dans cette légende se réfèrent à la nomenclature utilisée dans l'exemple 7.
01 HNF-1 LB sans extrait nucléaire
02 HNF-1 LB +1µg extrait nucléaire de foie
03 HNF-1 C1 sans extrait nucléaire
04 HNF-1 C1 +1µg extrait nucléaire de foie
05 HNF-1 C1 non ligué sans extrait nucléaire
06 HNF-1 C1 non ligué +1µg extrait nucléaire de foie
07 HNF-1 C2 sans extrait nucléaire
08 HNF-1 C2 +1µg extrait nucléaire de foie
09 HNF-1 C2 non ligué sans extrait nucléaire
10 HNF-1 C2 non ligué +1µg extrait nucléaire de foie
11 HNF-1 C3 sans extrait nucléaire
12 HNF-1 C3 +1µg extrait nucléaire de foie
13 HNF-1 C3 non ligué sans extrait nucléaire
14 HNF-1 C3 non ligué +1µg extrait nucléaire de foie

HNF-1C1, C2, C3 représentent 3 préparations différentes de l'oligonucléotide sens HNF-1 C.

### Exemple 1

### Obtention d'oligonucléotides fermés comportant une région d'autoappariement au moyen de la ADN ligase de T4.

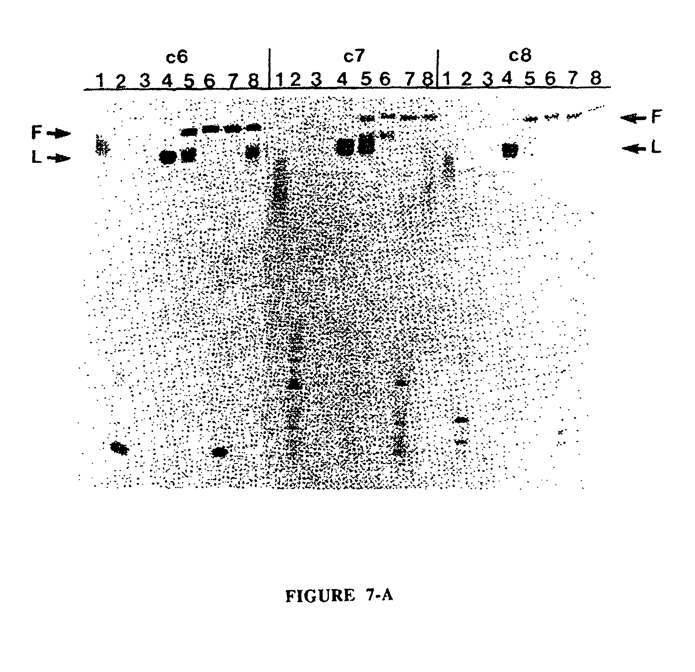

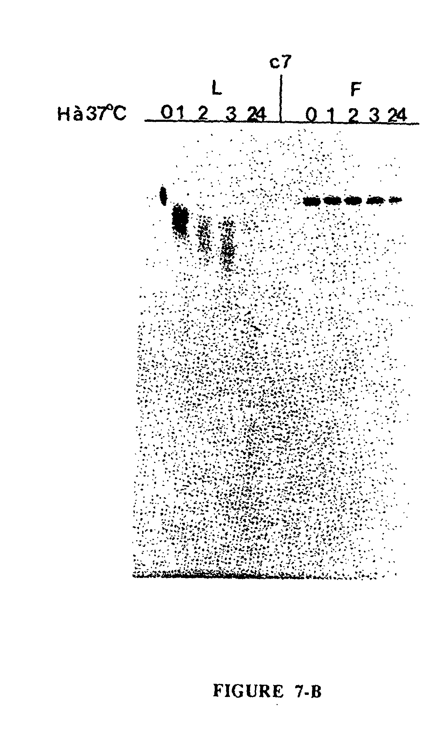

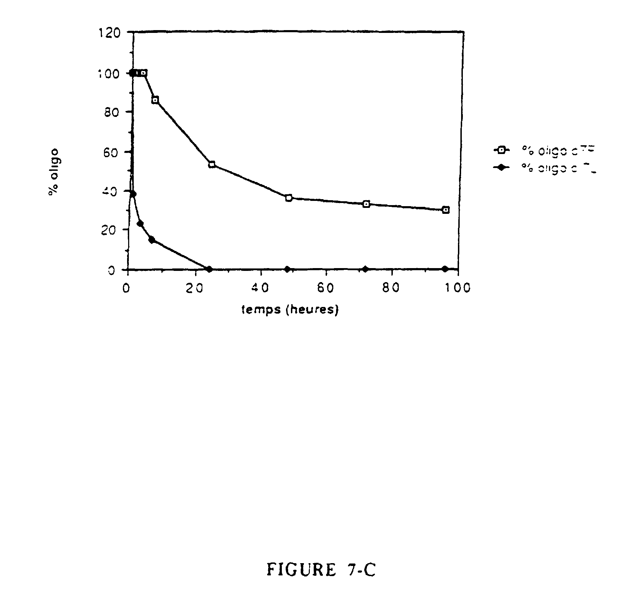

Les conditions expérimentales permettant la fermeture/ligation efficace des oligonucléotides dont les séquences sont données dans la Figure 7A sont les suivantes: 11 µM d'oligonucléotide linéaire marqué en 5' avec du g ³²P ATP (150 µg; activité spécifique= 2-3 x10 5 cpm/µg), 50 mM Tris-HCl pH 7.8, 10 mM MgCl₂, 20 mM DTT, 1 mM BSA sur M ATP et 10.000 unités de l'ADN ligase de T4 (2.000 u/µl; New England Biolabs) dans un volume total de 1 ml. Après incubation pendant 48 heures à 4°C., les mélanges de réaction sont extraits au phénol/chloroforme/alcool isoamilique, précipités à l'ethanol absolu, lavés à l'ethanol 80% et séchés. Les produits de ligation sont ensuite séparés de l'oligonucléotide non ligué par éiectrophorèse dénaturante en gel de polyacrylamide 20%/7 M urée. Les positions des oligonucléotides peuvent être observées directement par interférence de fluorescence en irradiant à 212 nm le gel placé sur une plaque contenant un chromophore fluorescent aux ultraviolets, ou bien par autoradiographie. L'oligonucléotide monomérique ligué se caractérise par une migration ralentie par rapport à son homologue non ligué (Figure 7A et B). Les bandes correspondantes à l'oligonucléotide monomérique fermé et à son homologue linéaire non ligué sont excisées du gel, et l'ADN est isolé par les techniques classiques d'extraction de gels de polyacrylamide. Dans le cas des séquences considérées , le rendement de formation de l'oligonucléotide monomérique fermé est de l'ordre de 65 à 75%.
Alternativement, les produits de la réaction de ligation peuvent êtres analysés sans purification préalable, directement après inactivation de la ADN ligase en chauffant le milieu de ligation pendant 2 minutes à 90°C

### Exemple 2

### Résistance des oligonucléotides circulaires en série désoxyribo- aux nucléases et aux phosphatases

Nous avons comparé la résistance des oligonucléotides circulaires et linéaires à l'action de la phosphatase alcaline (phosphomonoestérase hydrolysant les phosphates 3' et 5' de l'ADN et de l'ARN), de l'exonucléase VII (exodésoxyribonucléase digérant l'ADN monocaténaire depuis les deux extrémités 3' et 5'), et de la phosphodiestérase I (exonucléase digérant l'ADN.ou l'ARN à partir du 3' OH) Pour cette expérience, les oligonucléotides 5' ³²P dont les séquences sont indiquées dans la Figure 7A ont été préparés comme décrit ci-dessus (paragraphe 1). Après ligation, les mélanges réactionnels ont été chauffés pendant 2 minutes à 90°C afin d'inactiver l'ADN ligase.
1 µg d'oligonucléotide provenant de la réaction de ligation ou 1 µg de l'oligonucléotide linéaire homologue, non-circularisé et utilisé à titre de témoin, ont été incubés à 37°C dans un volume de 10 µl de 50 mM Tris HCl pH 7.5, 10 mM MgCl₂, 20 mM DTT en présence de 1 unité de phosphatase intestinale de veau (CIP) ou de 1 unité d'exonucléase VII de *E.coli* pendant 1 heure, ou de 5 x 10⁻⁵ unités de phosphodiestérase I de *Crotalus durissus* pendant 10 minutes. Après incubation, les produits de la réaction sont analysés sur gel de polyacrylamide 15% dans des conditions dénaturantes. Le résultat du gel est présenté en Figure 7A. On observe sur l'autoradiographie de ce gel que:
- les oligonucléotides fermés circulaires sont résistants à la phosphatase alors que les oligonucléotides linéaires y sont sensibles;
- les oligonucléotides fermés circulaires sont résistants à l'action de l'exonucléase VII alors que les oligonucléotides linéaires y sont sensibles;
- les oligonucléotides fermés circulaires sont résistants à l'action de la phosphodiestérase I alors que les oligonucléotides linéaires y sont sensibles.

Cette expérience montre que les oligonucléotides préparés comme indiqué plus haut sont bien des molécules circulaires fermées de façon covalente, et que ces molécules présentent une résistance totale aux enzymes exonucléolytiques.

### Exemple 3

### Résistance des oligonucléotides circulaires fermés aux nucléases sériques

Les oligonucléotides fermés circulaires présentent une résistance à la dégradation nucléolytique supérieure à celle des oligonucléotides linéaires lorsqu'ils sont incubés en présence de sérum.
1 µg d'oligonucléotide fermé et 1 µg d'oligonucléotide linéaire correspondant, préparés et purifiés après ligation comme décrit dans le paragraphe 1, sont incubés à 37°C dans 10 µl de milieu DMEM contenant 10% de sérum de veau foetal. La cinétique de la réaction est suivie jusqu'à 96 heures, et les prélèvements effectués en fonction du temps analysee sur gel de polyacrylamide 15% en conditions dénaturantes.
L'autoradiographie d'un gel d'analyse de la dégradation sur 24 heures est présenté en Figure 7B.
La figure 7-C est la représentation graphique d'une étude sur 96 heures de la dégradation d'un oligonucléotide linéaire (c7L) en comparaison avec la dégradation d'un oligonucléotide circulaire (c7F) dans les conditions décrites ci-dessus. Ce graphique met en évidence les différences considérables de stabilités entre ces deux types d'oligonucléotides.
Ces expériences permettent de tirer les conclusions suivantes:
- les oligonucléotides linéaires sont rapidement dégradés par les nucléases du sérum. La dégradation intervient dès les premières minutes de l'incubation, et est complète après quelques heures;
- le temps de demie-vie d'un oligonucléotide linéaire normal, non modifié, est très inférieur à 1 heure, cette durée pouvant varier légèrement en plus ou en moins selon les sérums utilisés;
- la dégradation des oligonucléotides linéaires est processive, et on observe l'apparition de produits de dégradation de longueur décroissante, devenant de plus en plus courts en fonction du temps, ce qui indique que la dégradation est principalement le fait d'exonucléases, et en particulier de 3'. exo;
- en revanche, les oligonucléotides fermés sont résistants à la nucléolyse par les enzymes sériques, et moins de 60% de conversion en produits de dégradation est observée même après 96 heures (4 jours) d'incubation à 37°C.
- le temps de demie-vie d'un oligonucléotide circulaire fermé dans le sérum est très supérieur à 24 heures.

Ces résultats confirment que la dégradation des oligonucléotides antisens dans le sérum est principalement le fait d'exonucléases et non pas d'endonucléases et mettent en évidence la résistance à la dégradation des oligonucléotides fermés.

Des oligonucléotides fermés naturels, ne portant pas de modifications chimiques particulières, présentent une résistance aux nucléases sériques similaire à la résistance décrite pour des dérivés modifiés. Cette expérience montre donc que dans des condition standards d'incubation d'oligonucléotides en présence de sérum, les oligonucléotides fermés qui font l'objet de ce brevet présentent un avantage notable sur les oligonucléotides linéaires.

### Exemple 4

### Hybridation d'oligonucléotides fermés en série désoxyribo- avec de l'ADN et de l'ARN

Pour présenter un effet antisens, les oligonucléotides antisens doivent pouvoir hybrider avec leur cible dans des conditions de stabilité satisfaisante. Nous avons analysé l'hybridation entre des oligonucléotides fermés et des polynucléotides portant des séquences complémentaires, soit en série ribo- soit en série désoxyribo-.

### Exemple 4-1

### Mise en évidence de l'hybridation entre un oligonucléotide fermé et un ADN linéaire

On incube 300 ng d'oligonucléotide circulaire fermé, préparé et purifié comme décrit ci-dessus, -ou 160 ng de l'oligonucléotide homologue linéaire à titre de témoin- avec un oligomer froid long de 42 nucléotides comportant une séquence de 21 nucléotides complémentaire aux 21 bases formant la grande boucle dans la structure fermée (voir la séquence de la Figure 6C) Les rapports molaires entre l'oligonucléotide marqué et l'oligonucléotide complémentaire froid varient de 10:1 à 1:5 L'hybridation se fait pendant 1 heure à 37°C dans 1X SSC (150 mM NaCl, 15 mM Na₃citrate, pH 7.0). Après incubation , les produits sont analysés sur gel de 20% polyacrylamide non dénaturant. Les résultats de l'autoradiographie du gel sont présentés en Figure 8A.
On observe sur ce gel que les cinétiques de déplacement des bandes entre les positions monocaténaire et bicaténaire sont identiques pour les oligonucléotides linéaires et les oligonucléotides fermés. Ce résultat signifie que les efficacités d'hybridation entre un oligonucléotide fermé et un ADN complémentaire sont identiques à celles d'un oligonucléotide linéaire avec une séquence complémentaire.

### Exemple 4-2

### Cartographie de l'hybridation entre un oligonucléotide fermé et un ARN linéaire

L'expérience suivante consiste à analyser par la technique de protection à la nucléase S1 la position des régions bicaténaires lors de l'hybridation entre un desoxyribonucléotide fermé et un oligoribonucléotide comportant une séquence qui est complémentaire à la région en boucle de l'oligonucléotide fermé. La nucléase S1 est une endonucléase spécifique des acides nucléiques simple brin, qui ne digère que les séquences non appariées. C'est un enzyme qui permet de cartographier les régions qui sont sous forme bicaténaire et de les différencier des régions monocaténaires.
On incube un oligonucléotide fermé, synthétisé et purifié comme décrit plus haut, avec un ARN linéaire long de 43 nucléotides, comportant une séquence de 27 bases complémentaires aux 21 nucléotides de la boucle de l'oligonucléotide fermé et à 6 des nucléotides engagés dans la région auto-appariée (voir la séquence de la Figure 6C). Cet ARN a été transcrit par l'ARN polymérase de T7 à partir d'une matrice synthétique et marqué avec de l'a³²P ATP à une activité spécifique élevée (2,3 x 10⁸ cpm/µg). Après transcription l'ARN transcrit est purifié par électrophorèse sur gel de polyacrylamide 15%/7 M urée, elué du gel et précipité à l'éthanol, puis suspendu dans de l'eau.
Après incubation pendant 1 heure à 37°C de 300 ng d'oligonucléotide fermé ou de son homologue linéaire avec 1 ng d'ARN dans 0.15 M NaCl, 0.1 M Hepes pH 7.9, 0.3 mM EDTA, on dilue le mélange de réaction 10 fois dans 50 mM NaCl, 33 mM acétate de sodium pH 4.4 et 30 µM ZnSO₄ et on ajoute 4 unités de nucléase S1. On prélève des aliquotes après 3 minutes et 30 minutes de digestion à 37°C et on analyse les produits obtenus sur gel de polyacrylamide 20% dans des conditions dénaturantes. A titre de contrôle, on a également incubé dans des conditions identiques l'ARN radioactif en présence d'un oligonucléotide linéaire complémentaire à l'ARN sur 37 nucléotides.
Les résultats de cette expérience sont présentés en Figure 8B
On observe sur ce gel que:
- L'ARN incubé en l'absence d'oligonucléotide complémentaire ou en présence d'un oligonucléotide non complémentaire, est effectivement non hybridé et complètement digéré par la nucléase S1;
- l'ARN incubé avec un oligonucléotide linéaire contrôle est protégé de la digestion sur une longueur de 37 nucléotides, ce qui correspond à la longueur des séquences complémentaires; après 30 minutes d'incubation le profil de protection se déplace vers des bandes centrées autour de deux bandes majoritaires de 35 et 34 nucléotides, respectivement ce qui peut correspondre à une "respiration" de la molécule bicaténaire;
- l'ARN incubé avec l'oligonucléotide fermé présente un profil de protection caractéristique, avec plusieurs bandes réparties selon une distribution centrées autour d'une bande de longueur de 27 nucléotides correspondant à la bande majoritaire; après 30 minutes d'incubation les bandes protégés majoritaires se situent au niveau de 20 et 21 nucléotides;
- le profil de protection observé lors de l'incubation de l'ARN avec un oligonucléotide de même séquence que l'oligonucléotide circularisé, mais linéaire est identique à celui de l'oligonucléotide circularisé;
- le schéma de protection de l'ARN par l'oligonucléotide fermé montre donc que l'hybridation entre l'ARN et la boucle circulaire de l'oligonucléotide s'effectue sur une longueur optimale de 21 nucléotides, comprenant ainsi la totalité de la boucle. L'ARN complémentaire peut même déplacer les nucléotides appariés dans la partie bicaténaire de l'oligonucléotide pour s'y hybrider.

Cette expérience démontre que l'hybridation entre un oligonucléotide fermé et un ARN comportant une région complémentaire à la boucle peut se réaliser dans des conditions de température et de force ionique standard et que l'hybride ainsi formé présente les caractéristique normales d'une molécule bicaténaire résistante à la nucléase S1.

### Exemple 5

### Activation de l'activité RNase H par un hybride formé entre un oligonucléotide circularisé fermé et un ARN linéaire

On sait que les effets antisens d'oligonucléotides complémentaires à un ARN messager sont, dans de nombreux cas, le résultat d'une action de la RNase H cellulaire sur le substrat ainsi formé. La RNase H est une activité enzymatique qui dégrade l'ARN lorsqu'il se trouve sous la forme d'un hybride ARN/ADN. Nous avons donc vérifié que l'hybridation entre un oligonucléotide antisens fermé et un ARN linéaire créait bien un substrat pour la RNase H.
La structure de l'ARN linéaire utilisé pour cette expérience ainsi que sa préparation ont été décrites au paragraphe ci-dessus.
L'hybridation de 300 ng d'oligonucléotide fermé ou de son homologue linéaire avec 1 ng d'ARN radioactif (2,3 x 10⁸ cpm/µg) comportant une région complémentaire à la partie en boucle de l'oligonucléotide antisens est réalisée dans les conditions décrites dans le paragraphe 4-2. Ces conditions assurent une hybridation de la totalité de l'ARN à l'ADN complémentaire. On dira que la réaction d'hybridation est "conduite" par l'ADN. Après incubation, on dilue 10 fois le volume, en ajustant le tampon d'incubation à 20 mM Tris HCl pH 7.5, 100 mM KCl, 10 mM MgCl₂, 0.1 mM DTT, et on ajoute 2 unités de RNase H. On incube à 37°C pendant 20 minutes et on analyse les produits de la réaction comme ci-dessus. Les résultats du gel sont présentés dans la Figure 8C. On observe sur ce gel que:
- l'ARN marqué non hybridé avec une séquence d'ADN complémentaire est complètement résistant à l'action de la RNase H;
- l'ARN marqué, long de 43 nucléotides, hybridé avec un oligonucléotide linéaire contrôle complémentaire à 37 bases de l'ARN devient partiellement sensible à l'action de la RNase H, et donne des produits de dégradation;
- l'ARN marqué hybridé avec un oligonucléotide fermé, dont la boucle est complémentaire à 21 nucléotides de cet ARN, devient très sensible à l'action de la RNase H et donne une série de produits de dégradation dont les longueurs sont compatibles avec les analyses de protections à la S1 décrites dans le paragraphe ci-dessus.

En fait l'expérience d'induction de substrat pour la RNase H est l'image en miroir de l'expérience de protection à la S1 et les résultats obtenus dans les deux cas sont tout à fait cohérents, indiquant à la fois des positions d'hybridations similaires et montrant qu'un substrat pour la RNase H peut être créé par hybridation entre un ARN linéaire et un oligodésoxyribonucléotide circulaire.

Cette expérience montre donc qu'un ARN linéaire hybridé partiellement à un oligodésoxyribonucléotide fermé devient un substrat pour la RNase H, ce qui implique qu'un ADN circulaire hybridé à un ARN messager cible peut exercer un effet antisens par le biais de l'action de cet enzyme sur un substrat ainsi généré. De plus, cette expérience montre également que, dans des conditions expérimentales de concentration identiques, le substrat ARN/ADN circulaire donne lieu à une dégradation plus importante par la RNase H que le substrat ARN/ADN linéaire. Dans des conditions identiques, toutes choses étant égales par ailleurs, les oligodésoxyribonucléotides antisens circulaires présentent donc un avantage supplémentaire sur les oligonucléotides antisens linéaires.

### EXEMPLE 6

### Inhibition de la multiplication du virus Herpès Simplex type 1 (HSV-1) par des oligonucléotides antisens circulaires.

L'oligonucléotide GT, dont la séquence est donnée ci-dessous a été synthétisé, circularisé ou non, et utilisé dans les expériences décrites ici, soit sous forme linéaire, soit sous forme circulaire.

### 1-Protocoles expérimentaux

### Séquence et circularisation de l'oligonucléotide GT

Séquence de GT: 5' GTG GGA CGT TCC TCC TGC GGG AAG CGG C 3'

Pour permettre une circularisation chimique efficace, l'oligonucléotide GT a été synthétisé sous forme 3'P, et circularisé en positionnant les extrémités 5' et 3'P par hybridation avec l'oligonucléotide de séquence partiellement complémentaire suivante: 5' CCA CGC CG 3'

Les conditions de ligation utilisées sont les suivantes:
pour 100 µg d'oligonucléotide GT : 100µg oligonucléotide complémentaire
   0,25 M MES, pH 7,4
   20 mM MgCl₂
   0,2 M CnBr
Volume réactionnel: 500 µl
Incubation à 4°c pendant 30 minutes. La réaction est arrêtée par addition de 1/10 de volume d'acétate de sodium 3M, et 2,5 volumes d'ethanol absolu, les oligonucléotides précipités et purifiés sur gel de polyacrylamide dénaturant.

### Infection des cellules en présence ou en absence d'oligonucléotides antisens

Les cellules (cellules Vero ATCC -passage 121- cultivées en milieu (Gibco) MEM complémenté de 5 ou 10% de SVF, de L-glutamine, d'acides aminés non essentiels et de pénistrepto) sont repiquées la veille de l'infection à une densité de 5.10⁴ cellules par puits de 2 cm². 16 à 24 heures après,les cellules sont infectées avec RSV1F à une multiplicité d'infection de 3 pfu/cellule en présence ou en absence d'oligonucléotides.
Les oligonucléotides sont dilués dans du milieu de culture sans sérum en concentration 2X par rapport à la concentration finale désirée pour être ajoutés sous un volume de 50 µl. Le virus est ajouté également sous un volume de 50 µl, 5 mn après les oligonucléotides. Les cellules sont donc traitées sous un volume de 100µl (50 µl oligonucléotides + 50 µl virus) pendant une heure à 37°C avec agitation douce toutes les 15 mn. Alternativement, les oligonucléotides peuvent être ajoutés plusieurs heures avant l'infection.
Après 1 heure d'incubation, le milieu est aspiré et 500 µl de milieu complet sont ajoutés sur les cellules. L'incubation est poursuivie 24 h avant d'être arrêtée par congélation des plaques dans l'azote liquide.
Toutes les mesures d'inhibition sont effectuées en duplicats ou triplicats.

### Titration du virus

Les virus sont récupérés directement dans le milieu de culture après 3 cycles rapides congélation azote liquide/décongélation à 37°C. Ils sont ensuite dilués en milieu sans sérum pour effectuer la titration proprement dite.
Les cellules indicatrices sont repiquées la veille en milieu complet à raison de 10⁵ cellules/puits de 2 cm².
Le lendemain, le milieu est aspiré et on dépose 100 µl ces différentes dilutions/puits. Après une incubation d'une heure à 37°C avec agitation toutes les 15 mn, le milieu est aspiré et les cellules sont recouvertes avec du milieu complet contenant 1,2% de méthyl cellulose (concentration finale de sérum 2,5%) pendant 3 jours à 37°C.
Après 3 jours, le milieu est éliminé, les cellules sont fixées avec du PBS-10% formol (solution à 37%) pendant 20 mn puis colorées au cristal violet 2% (dans PBS-20% éthanol) pendant 20 mn. Les plaques sont ensuite rincées et les plages sont comptées par transparence sur le négatoscope.
Les titration sont effectuées en double pour chaque point.
Les calculs d'inhibitions sont effectuées par rapport aux titres viraux observés en l'absence d'oligonucléotide.

### 2-Résultats

### -Analyse des effets d'oligonucléotides circulaires sur la croissance cellulaire

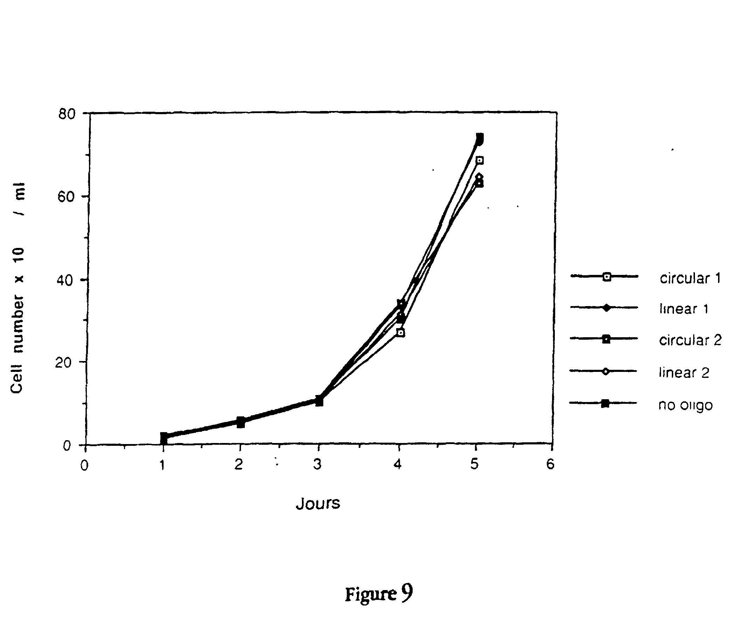

La figure 9 présente les résultats de mesure de croissance cellulaire (cellules Vero) en présence d'oligonucléotides divers, linéaires ou circulaires, en comparaison avec la croissance normale des cellules seules. Dans cette expérience, les oligonucléotides étaient utilisés à une concentration de 20µM.
Les résultats montrent que les courbes de croissance sont superposées. Les oligonucléotides linéaires ou circulaires ne semblent présenter aucun effet toxiques sur la croissance cellulaire.

### -Analyse des effets d'oligonucléotides circulaires sur la multiplication de HSV-1

Dans cette expérience, on a comparé les effets d'oligonucléotides antisens GT (dont la séquence est donnée ci-dessus) sous forme linéaire, ou sous forme circulaire. Deux conditions différentes d'inhibition ont été comparés.

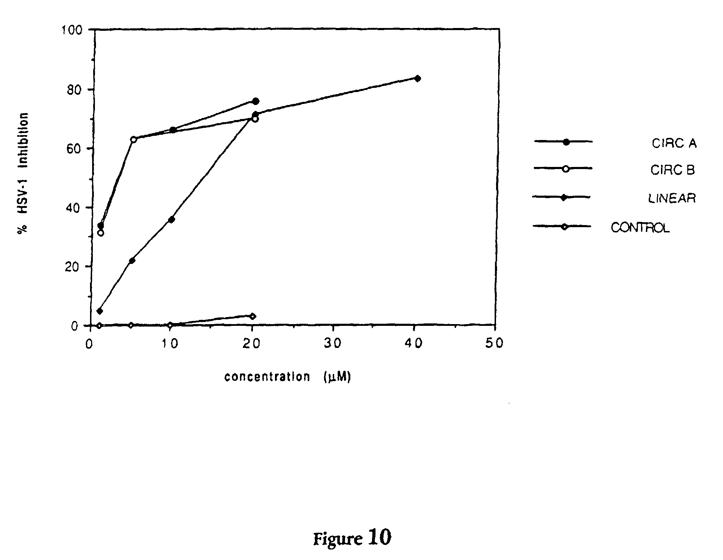

En A, les antisens sont ajoutés au moment de l'infection, alors qu'en B, ils sont introduit dans le milieu 4 heures avant l'infection. Les résultats présentés en figure 10 montrent que des oligonucléotides antisens circulaires inhibent la multiplication virale. L'inhibition est de 30% à 2 µM, et atteint 65% à 5µM. A ces basses concentrations, les oligonucléotides circulaires présentent un effet d'inhibition supérieur à celui de l'oligonucléotide linéaire.

### EXEMPLE 7

### Propriétés des oligonucléotides de type "sens"

### 1-Obtention des oligonucléotides de type sens

Les conditions expérimentales permettant la ligation efficace de l'oligonucléotide dont la séquence est donnée dans la figure 5-B sont similaires à celles décrites dans l'exemple 1.
Les produits de ligation sont ensuite analysés, par électrophorèse dénaturante en gel de polyacrylamide de 12%/7M urée.
Pour la préparation des oligonucléotides sens fermés marqués servant aux essais de gel de retard, les conditions sont les suivantes:
22n moles d'oligonucléotide sont marqué en 5' avec du gamma-32 ATP (activité spécifique 2-5x10⁸ cpm/µg), 50mM Tris HCl pH7,8, 10mM MgCl2, 20mM DTT, 1mM ATP, 1mM bSA et 400 unités de l'ADN ligase de T4 dans un volume de 10µl. L'incubation se fait pendant 2h à 16°C. Les produits de ligation sont purifiés par électrophorèse dénaturante en gel de polyacrylamide de 12%/7M urée, les bandes correspondantes à l'oligonucléotide fermé sont détectées par autoradiographie excisées du gel et l'ADN est isolé par les techniques classiques d'extraction de gels de polyacrylamide.
Alternativement, les produits de la réaction de la ligation sont utilisés sans purification préalable, après inactivation de l'ADN ligase par chauffage du milieu de ligation pendant 2 minutes à 90°C extraction au phénol/chloroforme/alcool isoamilique, précipitation à l'alcool absolu en présence de 20µg de glycogène comme entraîneur et lavage à l'alcool 80%

### 2-Résistance des oligonucléotides de type sens aux nucléases sériques

Les oligonucléotides "sens" présentent une résistance à la dégradation nucléolytique lorsqu'ils sont incubés en présence de sérum. Les comparaisons effectuées entre la résistance des oligonucléotides circularisés et des oligonucléotides bicaténaires linéaires donnent les mêmes résultats que ceux présentés dans la figure 7-C. Dans tous les cas, les oligonucléotides fermés présentent une résistance supérieure à celle des oligonucléotides non fermés. Le temps de demie-vie des oligonucléotides sens fermés est supérieur au moins d'un facteur 10 à celui des oligonucléotides linéaires bicaténaires non fermés, présentant des extrémités 3' et 5' libres.

3-Mise en évidence de la fixation du facteur de transcription HNF-1 par un oligonucléotide circulaire de type sens comportant la séquence de reconnaissance sous forme auto-appariée.

Les expériences suivantes ont été réalisées avec les oligonucléotides dont la séquence est rappelée ci-dessous:

HNF-1 NON LIGUE:

3' 5'

T
T T-G-T-G-T-G-G-T-T-A-A-T G-A-T-C-T-A-C-A-G-T-T-A- T T
T T
T-C-A-C-A-C-C-A-A-T-T-A—C-T-A-G-A-T-G-T-C-A-A-T- T
T T

HNF1-CIRCULAIRE (HNFI-C):

  T
T   T -G-T-G-T-G-G-T-T-A-A-T-G-A-T-C-T-A-C-A-G-T-T-A- T -T
                                                          T
 T -T -C-A-C-A-C-C-A-A-T-T-A-C-T-A-G-A-T-G-T-C-A-A-T- T -T

HNFI-LINEAIRE BICATENAIRE (HNF1-LB):

-G-T-G-T-G-G-T-T-A-A-T-G-A-T-C-T-A-C-A-G-T-T-A-
-C-A-C-A-C-C-A-A-T-T-A-C-T-A-G-A-T-G-T-C-A-A-T-

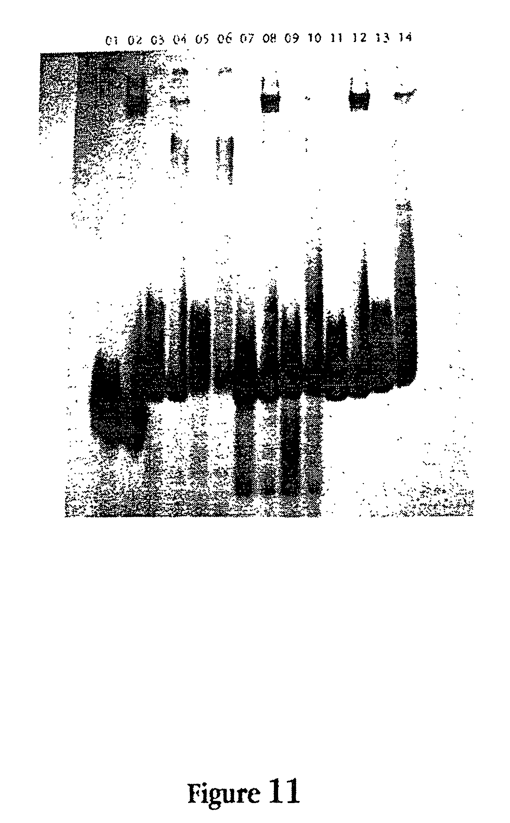

Nous avons comparé l'efficacité de fixation du facteur de transcription HNF1 (obtenu à partir d'extraits nucléaires de foie) sur des oligonucléotides linéaire double brin, des oligonucléotides en épingle à cheveux, des oligonucléotides circulaires, non ligués ou refermés par l'action de la ligase de T4 (la séquence de l'oligonucléotide circulaire utilisé est donné en figure 5-B).
3 f moles de chaque oligonucléotide (activité spécifique 7000 cpm/f moles pour l'oligonucléotide linéaire double brin et 3500 cpm/f moles pour les autres oligonucléotides) sont incubés dans un volume final de 14µl avec 1µg d'un extrait protéique nucléaire de foie dans 10mM Hepes pH 7,9, 50mM KCl, 10% glycerol, 0,1mM EDTA, 0,5mM DTT, 0,5 mM PMSF, 6 mM MgCl2, 6mM spermine en présence de 1,5 µg de poly dI.dC-poly dI.dC et 250 ng d'ADN de sperme de saumon soniqué comme compétiteurs non spécifique. Après 10 mn à 4°C, les mélanges de réaction, ainsi que des contrôles dans lesquels on a omis l'addition des protéines, sont déposés sur un gel natif de polyacrylamide 6%/0,25 x TBE. A la fin de la migration, le gel est fixé dans une solution 10% acide acétique, 10% métanol, transféré sur du papier 3MM, séché et autoradiographié. Le résultat de cette expérience est présenté dans la figure 11.
On observe que:
1. L'affinité de fixation du facteur de transcription, pour l'oligonucléotide circulaire fermé est similaire à la fixation observée sur les oligonucléotides linéaire double brin ( ou en épingle à cheveux, résultats non présentés ici). De telles structures peuvent donc être utilisées comme agent pour fixer des facteurs de transcription, des transactivateurs, ou tous autres protéines se fixant sur une séquence d'ADN spécifique.
2. La fixation du facteur de transcription HNF1 sur l'oligonucléotide circulaire non ligué est de 5 à 10 fois inférieure à celle observée sur l'oligonucléotide circularisé par action de la ligase. Le site de ligation dans l'oligonucléotide sens est centré dans la portion bicaténaire et correspond à l'axe du pseudopalindrome qui constitue le site de fixation du dimer de HNF1. La présence d'un "nick" déstabilise donc la liaison ADN/protéine. Ce résultat confirme la nature fermée de l'oligonucléotide circulaire "sens" traité par la ligase de T4.

### REFERENCES BIBLIOGRAPHIQUES CITEES DANS LE TEXTE

Andrus, A.; Geiser, T.; Zon, G. (1989), *Nucleosides Nucleotides,* **8,** 5-6, 967-8
Anfossi, Giovanni; Gewirtz, Alan M.; Calabretta, Bruno, (1989), *Proc. Natl. Acad. Sci. U.S.A*., **86,** 3379-83
Barbato, S., De Napoli, L., Mayol,L, Piccialli, G, and Santacroce, C. (1989). *Tetrahedron*, 54, 4523-4536
Bazile D; Gautier C; Rayner B; Imbach JL; Paoletti C; Paoletti J, (1989), *Nucleic Acids Res,* **17,** 7749-59
Bertrand JR; Imbach JL; Paoletti C; Malvy C, (1989), *Biochem Biophys Res Commun,* 164, 311-8
Cameron FH; Jennings PA, (1989), *Proc Natl Acad Sci U S A,* **86**, 9139-43
Caruthers, M.H. (1985) *Sciences,* 230, 281
Cazenave C; Chevrier M; Nguyen TT; Helene C, (1987), *Nucleic Acids Res,* **15,** 10507-10521
Cazenave, C.; Stein, C. A.; Loreau, N.; Thuong, N. T.; Neckers, L. M.; Subasinghe, C.; Helene, C.; Cohen, J. S.; Toulme, J. J., (1989), *Nucleic Acids Res.,* **17,** 4255-73
Chang, E. H.; Yu, Z.; Shinozuka, K.; Zon, G.; Wilson. W. D.; Strekowska, A., (1989), *Anti-Cancer Drug Des.,* **4,** 221-32 Cope, F. O.; Wille, J. J., (1989), *Proc. Natl. Acad. Sci.* U.S.A., **86,** 5590-4
Cotten M. and Birnstiel ML. (1989). *EMBO J.* **8,** 3861-3866.
Cotten M; Schaffner G; Birnstiel ML, (1989), *Mol Cell Biol,* **9,** 4479-87
Degols, G.; Leonetti, J.P.; Gagnor, C.; Lemaitre, M.; Lebleu, B., (1989), *Nucleic Acids Res.,* **17,** 9341-50
DDe Vroom E., Broxterman, H.J., Sliedregt, L.A.J., Van der Marel, G.A., Van Boom, J.H., (1988), *Nucl. Ac. Res.,* 16, 4607-4620
Dervan PB, (1986), *Science,* **232,** 464-71
Durand M; Maurizot JC; Asseline U; Barbier C; Thuong NT; Helene C, (1989), *Nucleic Acids Res,* **17,** 1823-1837
Fedor MJ; Uhlenbeck OC, (1990), *Proc Natl Acad Sci*, **87,** 1668-72
Francois JC; Saison-Behmoaras T; Barbier C; Chassignol M; Thuong NT; Helene C, (1989), *Proc Natl Acad Sci*, **86**, 9702-9706
Francois JC; Saison-Behmoaras T; Chassignol M; Thuong NT; Helene C, (1989), *J Biol Chem*, **264** 5891-5898
Francois JC; Saison-Behmoaras T; Helene C, (1988), *Nucleic Acids Res,* **16**, 11431-11440
Francois JC; Saison-Behmoaras T; Thuong NT; Helene C, (1989), *Biochemistry*, **28**, 9617-9619
Froehler, B.C., Ng, P. and Matteucci, M.D. (1986) Nucl. Acid Res., 14, 5399
Gagnor, C., Bertrand, J.R., Thenet, S., Lemaitre, M., Morvan, F., Rayner, B., Malvy, C., Lebleu, B., Imbach, J.L., Paoletti, C., (1987), *Nucleic Acids Res*., **15,** 10419-36
Gao, W., STein, C.A., Cohen, J.S., Dutchmann, G. and Cheng, Y.C., (1988). *J. Biol. Chem*., **264,** 11521-11532
Goodchild, John; Agrawal, Sudhir; Civeira, Maria P.; Sarin, Prem S.; Sun, Daisy; Zamecnik, Paul C., (1988), *Proc. Natl. Acad. Sci. U.S.A*., **85,** 5507-11
Haseloff J; Gerlach WL, (1988), Nature, **334,** 585-91
Helene C, (1989) *Br J Cancer,* **60,** 157-60
Helene C; Thuong NT, (1988), *Biochem Pharmacol,* **37,** 1797-1798 Jeffries AC; Symons RH, (1989), *Nucleic Acids Res,* **17,** 1371-7
Jessus C; Chevrier M; Ozon R; Helene C; Cazenave C, (1989), *Gene*, **72,** 311-312
Kabanov, A. V.; Vinogradov, S. V.; Ovcharenko, A. V.; Krivonos, A. V.; Melik-Nubarov, N. S.; Kiselev, V. I.; Severin, E. S., (1990), *FEBS Lett.,* **259**, 327-30 Le Doan T; Chavany C; Helene C, (1989) *Bull Cancer,* **76,** 849-52
Leonetti, J.P, Degols, G., Milhaud, P., Gagnor, C., Lemaitre, M., Lebleu, B., (1989), *Nucleosides Nucleotides*, **8**, 5-6, 825-8
Maher, Louis J, III; Dolnick, Bruce J., (1988), *Nucleic Acids Res.,* **16**, 3341-58 Marcus-Sekura, Carol J.; Woerner, Amy M.; Shinozuka, Kazuo; Zon, Gerald; Quinnan, Gerald V., Jr., (1987), *Nucleic Acids Res.,* **15,** 5749-63
Matsukura, Makoto; Zon, Gerald; Shinozuka, Kazuo;Robert-Guroff, Marjorie; Shimada, Takashi; Stein, C. A.; Mitsuya, Hiroaki; Mitsuya, Hiroaki; Wong-Staal, Flossie; et al., (1989), *Proc. Natl. Acad. Sci. U.S.A*., **86,** 4244-8
Miroshnichenko, O. I.; Ponomareva, T. I.; Tikhonenko, T. I., (1989), *Gene*, **84,** 83-9
Perbost M; Lucas M; Chavis C; Pompon A; Baumgartner H; Rayner B; Griengl H; Imbach JL, (1989), *Biochem Biophys Res Commun,* **165**, 742-7
Perroualt, L., Asseline, U., Rivalle,C., Thuong, N.Y., Bisagni, E., Giovanelli, C., LeDoan, T. and Helene, C. (1990). *Nature,* **344,** 358-361
Sampson, J. R.; Sullivan, F. X.; Behlen, L. S.; DiRenzo, A. B.; Uhlenbeck, O. C., (1987), *Cold Spring Harbor Symp. Quant. Biol*., **52,** 267-75
Sankar, Sabita; Cheah, Keat Chye; Porter, Alan G., (1989), *Eur. J. Biochem*. **184**, 39-45
Sarver, N., Cantin, E.M., Pairoj, S., C., Zaia, J.A., Ladne, P.A., Stephens, D. A. and Rossi, J.J. (1990). *Science*, **247,** 1222-1225.
Sheldon CC; Symons RH, (1989), *Nucleic Acids Res*, **17,** 5679-85 Sheldon CC; Symons RH, (1989), *Nucleic Acids Res,* **17,** 5665-77
Shuttleworth, John; Matthews, Glenn; Dale, Les; Baker, Chris; Colman, Alan, (1988), *Gene*, **72**,1-2, 267-75
Stevenson, Mario; Iversen, Patrick L., (1989), J. *Gen. Virol*., **70,** 2673-82
Sun JS; Asseline *U;* Rouzaud D; Montenay-Garestier T; Nguyen TT; Helene C, (1987), *Nucleic Acids Res,* **15,** 6149-58
Sun JS; Francois JC; Lavery R; Saison-Behmoaras T; Montenay-Garestier T; Thuong NT; Helene C, (1988), *Biochemistry*, **27,** 6039-6045
Sun JS; Francois JC; Montenay-Garestier T; Saison-Behmoaras T; Roig V; Thuong NT; Helene C, (1989) *Proc Natl Acad Sci* **86,** 9198-9202
Symons RH, (1989), *Trends Biochem Sci*, **14,** 445-50
Symons RH; Hutchins CJ; Forster AC; Rathjen PD; Keese P; Visvader JE, (1987), *J Cell Sci Suppl*, **7**, 303-18
Tortora, Giampaolo; Clair, Timothy; Cho-Chung, Yoon Sang, (1990), *Proc. Natl. Acad. Sci. U.S.A.,* **87,** 705-8
Toulme JJ; Krisch HM; Loreau N; Thuong NT; Helene C, (1986), *Proc Natl Acad Sci*, **83,** 1227-31
Trung Le Doan; Perrouault L; Chassignol M; Nguyen TT; Helene C, (1987), *Nucleic Acids Res,* **15,** 8643-8659
Uhlenbeck, Olke C., (1987), *Nature,* **328,** 596-600
Uhlenbeck, Olke C.; Dahm, Sue Ann C.; Ruffner, Duane E.; Fedor, Martha J., (1989), *Nucleic Acids Symp. Ser*., 21, 95-6 Vasseur, M. (1990), *Biofutur*, avril 1990, 18-28.
Verspieren P; Cornelissen AW; Thuong NT; Helene C; Toulme JJ, (1987), *Gene*, **61,** 307-315
Vlassov, V.V., Zarytova, V.F., Kutiavin, I.V., Mamaev, S.V. and Podyminogin, M.A. (1986) *Nucl. Acids Res.,* **14,** 4065-4076.
Wang AH; Cottens S; Dervan PB; Yesinowski JP; van der Marel GA; van Boom JH, (1989), *J Biomol Struct Dyn,* **7,** 101-17
Westermann, P.; Gross, B.; Hoinkis, G., (1989), *Biomed. Biochim. Acta*, **48,** 85-93
Zerial A; Thuong NT; Helene C, (1987), *Nucleic Acids Res,* **15,** 9909-9919
Zheng, H., Sahai, Beni M., Kilgannon, P., Fotedar, A., Green, D. R., (1989), *Proc. Natl. Acad. Sci. U.S.A*., 86, 3758-62

## Revendications

1. Agent du type oligonucléotide constitué d' une ou plusieurs séquence(s) d'oligonucléotides monocaténaires, dont les extrémités sont reliées entre elles par des liens covalents pour former au moins partiellement une structure monocaténaire fermée, ledit agent étant **caractérisé en ce qu'**il est constitué d'au moins une séquence sélectionnée parmi les séquences suivantes :
a) un oligonucléotide antisens ;
b) un oligonucleotide sens, reconnu spécifiquement par une protéine naturelle.

2. Agent selon la revendication 1, **caractérisé en ce qu'**il est constitué d'un oligonucléotide antisens.

3. Agent selon la revendication 2 **caractérisé en ce qu'**il est constitué d'une séquence oligonucléotidique monocaténaire dont les extrémités 5' et 3' sont reliées par une structure covalente non nucléotidique.

4. Agent selon la revendication 2 , **caractérisé en ce qu'**il est constitué d'une séquence oligonucléotidique monocaténaire dont les extrémités 5' et 3' sont reliées entre elles par une liaison par une liaison nucléotidique.

5. Agent selon l'une des revendications 2 à 4 , **caractérisé en ce que** les séquences d'oligonucléotides ne comportent aucune extrémité 5' ou 3' libre.

6. Agent selon l'une des revendications 1 à 5, constitué d'un oligonucléotide antisens, **caractérisé en ce que** la ou les séquence(s) nucléotidique(s) du composé ne comportent pas de fragments susceptibles de s'auto-apparier.

7. Agent selon l'une des revendications 2 à 6, **caractérisé en ce que** au moins une séquence d'oligonucléotides est la séquence complémentaire d'une région d'ARN messager ou d'un fragment d'ADN naturel.

8. Agent selon l'une des revendications 1 à 7, **caractérisé en ce qu'**il comporte une partie polyribonucléotidique capable d'exercer une activité de clivage en trans sur un ARN.

9. Agent selon la revendication 1, **caractérisé en ce que** les séquences d'oligonucléotides comportent des fragments susceptibles de s'apparier pour fomer des auto-appariements bicaténaires.

10. Agent selon la revendication 9, **caractérisé en ce que** les fragments susceptibles de s'apparier peuvent être séparés par des fragments ne pouvant pas s'apparier.

11. Agent selon la revendication 10, **caractérisé en ce que** ladite protéine naturelle est un facteur de transcription.

12. Agent selon la revendication 11, **caractérisé en ce que** le facteur de transcription est HNF-1.

13. Agent selon la revendication 12, **caractérisé en ce que** les fragments susceptibles de s'apparier peuvent être séparés par des fragments ne pouvant pas s'apparier.

14. Agent selon l'une des revendications 2 à 11, **caractérisé en ce que** la ou les séquence(s) d'oligonucléotides peuvent comporter des nucléotides en série désoxyribo- ou en série ribo- naturelle ou non naturelle.

15. Agent selon l'une des revendications 1 à 11 et 14, **caractérisé en ce que** un composant ayant une activité biologique est greffé sur la structure monocaténaire fermée

16. Agent selon la revendication 15, **caractérisé en ce que** ledit composant est greffé sur la structure covalente non nucléotidique.

17. Agent selon l'une des revendications 1 à 11, 14-16, **caractérisé en ce que** la structure covalents non nucléotidique est une structure peptidique.

18. Agent selon l'une des revendications 1 à 11, 14-17, **caractérisé en ce que** la structure covalente non nucléotidique est une structure au moins partiellement lipidique.

19. Agent selon l'une des revendications 1 à 11, 14-18, **caractérisé en ce que** la séquence d'oligonucléotides est uniquement en série désoxyribo- ou en série ribo- naturelle ou non naturelle.

20. Agent selon l'une des revendications 1 à 11, 14-19, **caractérisé en ce que** le séquences nucléotidiques lui permettent d'adapter une structure bicaténaire, faisant intervenir des appariements anti-parallèles et/ou parallèles dans une conformation de type Moebius.

21. Agent selon l'une des revendications 1 à 11, 14-20, **caractérisé en ce que** les séquences d'oligonucléotides comportent entre 10 et 200 nucléotides.

22. Procédé de préparation d'un agent selon l'une des revendications 1 à 21, **caractérisé en ce que** l'on a effectué la synthèse chimique partielle ou totale de la chaîne monocaténaire du composé avant de le cycliser.

23. Procédé selon la revendication 22, **caractérisé en ce que** la séquence d'oligonucléotides est obtenue par une méthode biochimique.

24. Agent selon l'une des revendications 1 à 11, 14-21 pour la fabrication d'un médicament.

25. Utilisation d'un agent selon l'une des revendications 1 à 11, 14 à 21 pour la fabrication d'un médicament anti-viral ou anticancéreux

26. Agent selon la revendication 24, **caractérisée en ce que** le médicament est inducteur d'immuno-modulateurs naturels.

27. Agent selon la revendication 24, **caractérisée en ce que** le médicament est inducteur d'interférons.

28. Agent selon l'une des revendications 1 à 11, 14 à 21 pour la fabrication d'un médicament destiné à agir de façon spécifique sur la transcription ou la traduction de protéines dont on désire moduler le niveau d'expression.

29. Agent selon la revendication 28 comme agent de répression de l'expression de protéines directement impliquées dans les maladies de la prolifération et de la différenciation.

30. Utilisation d'un agent selon l'une des revendications 1 à 11, 14 à 21 à la fabrication d'un agent de diagnostic.

31. Utilisation selon la revendication 30, **caractérisée en ce que** l'agent est marqué.

32. Utilisation d'un agent selon l'une des revendications 1 à 11, 14 à 21 en cosmétique

33. Composition pharmaceutique **caractérisée en ce qu'**elle comporte à titre de principe actif au moins un agent selon l'une des revendications 1 à 11, 14-21.

34. Composition selon la revendication 33, **caractérisée en ce que** la composition pharmaceutique est sous une forme administrable par voie topique externe.

35. Composition selon la revendication 34, **caractérisée en ce qu'**il s'agit d'une composition anti-virale.

## Claims

1. Agent of the oligonucleotide type, which consists of one or more single-stranded oligonucleotide sequence(s) whose ends are linked to one another via covalent links to form at least partially a closed, single-stranded structure, the said agent being **characterized in that** it consists of at least one sequence selected from the following sequences:
a) an antisense oligonucleotide;
b) a sense oligonucleotide, specifically recognized by a natural protein.

2. Agent according to Claim 1, **characterized in that** it consists of an antisense oligonucleotide.

3. Agent as claimed in Claim 2, **characterized in that** it consists of a single-stranded oligonucleotide sequence whose 5' and 3' ends are linked via a non-nucleotide covalent structure.

4. Agent as claimed in Claim 2, **characterized in that** it consists of a single-stranded oligonucleotide sequence whose 5' and 3' ends are linked to one another via a nucleotide bond.

5. Agent as claimed in one of Claims 2 to 4, **characterized in that** the oligonucleotide sequences do not possess any free 5' or 3' end.

6. Agent according to one of Claims 1 to 5, consisting of an antisense oligonucleotide, **characterized in that** the nucleotide sequence(s) of the compound do(es) not contain fragments capable of self-pairing.

7. Agent according to one of Claims 2 to 6, **characterized in that** at least one oligonucleotide sequence is the sequence complementary to a region of messenger RNA or to a natural DNA fragment.

8. Agent according to one of Claims 1 to 7, **characterized in that** it contains a polyribonucleotide portion capable of exerting a trans cleavage activity on RNA.

9. Agent according to Claim 1, **characterized in that** the oligonucleotide sequences contain fragments capable of pairing to form double-stranded self-pairings.

10. Agent according to Claim 9, **characterized in that** the fragments capable of pairing can be separated by fragments incapable of pairing.

11. Agent according to Claim 10, **characterized in that** the said natural protein is a transcription factor.

12. Agent according to Claim 11, **characterized in that** the transcription factor is HNF-1.

13. Agent according to Claim 12, **characterized in that** the fragments capable of pairing can be separated by fragments incapable of pairing.

14. Agent according to one of Claims 2 to 11, **characterized in that** the oligonucleotide sequence(s) can contain nucleotides of the natural or unnatural deoxyribo- series or ribo- series.

15. Agent according to one of Claims 1 to 11 and 14, **characterized in that** a component having a biological activity is grafted onto the closed, single-stranded structure.

16. Agent according to Claim 15, **characterized in that** the said component is grafted onto the non-nucleotide covalent structure.

17. Agent according to one of Claims 1 to 11 and 14 to 16, **characterized in that** the non-nucleotide covalent structure is a peptide structure.

18. Agent according to one of Claims 1 to 11 and 14 to 17, **characterized in that** the non-nucleotide covalent structure is an at least partially lipid structure.

19. Agent according to one of Claims 1 to 11 and 14 to 18, **characterized in that** the oligonucleotide sequence is solely of the natural or unnatural deoxyribo- series or ribo- series.

20. Agent according to one of Claims 1 to 11 and 14 to 19, **characterized in that** the nucleotide sequences enable it to adopt a double-stranded structure involving antiparallel and/or parallel pairings in a Moebius type conformation.

21. Agent according to one of Claims 1 to 11 and 14 to 20, **characterized in that** the oligonucleotide sequences contain between 10 and 200 nucleotides.

22. Method for preparing an agent according to one of Claims 1 to 21, **characterized in that** the partial or total chemical synthesis of the single-stranded chain of the compound has been performed before the latter is cyclized.

23. Method according to Claim 22, **characterized in that** the oligonucleotide sequence is obtained by a biochemical method.

24. Agent according to one of Claims 1 to 11 and 14 to 21, for the manufacture of a medicament.

25. Use of an agent according to one of Claims 1 to 11 and 14 to 21, for the manufacture of an antiviral or anticancer medicament.

26. Agent according to Claim 24, **characterized in that** the medicament is an inducer of natural immunomodulators.

27. Agent according to Claim 24, **characterized in that** the medicament is an interferon inducer.

28. Agent according to one of Claims 1 to 11 and 14 to 21, for the manufacture of a medicament intended to act specifically on the transcription or translation of proteins whose level of expression it is desired to modulate.

29. Agent according to Claim 28, as an agent for repressing the expression of proteins directly involved in diseases of proliferation and of differentiation.

30. Use of an agent according to one of Claims 1 to 11 and 14 to 21, for the manufacture of a diagnostic agent.

31. Use according to Claim 30, **characterized in that** the agent is labelled.

32. Use of an agent according to one of Claims 1 to 11 and 14 to 21, in cosmetics.

33. Pharmaceutical composition, **characterized in that** it contains as active principle at least one agent according to one of Claims 1 to 11 and 14 to 21.

34. Composition according to Claim 33, **characterized in that** the pharmaceutical composition is in a form which can be administered by the external topical route.

35. Composition according to Claim 34, **characterized in that** it is an antiviral composition.

## Patentansprüche

1. Mittel vom Oligonukleotid-Typ, gebildet aus einer oder mehreren einzelsträngigen Oligonukleotidsequenz(en), deren Enden miteinander durch kovalente Bindungen verbunden sind unter Bildung von mindestens teilweise einer geschlossenen einzelsträngigen Struktur, wobei das Mittel **dadurch gekennzeichnet ist, dass** es aus wenigstens einer Sequenz gebildet wird, die ausgewählt ist unter den folgenden Sequenzen:
a) einem Antisinn-Oligonukleotid;
b) einem Sinn-Oligonukleotid, das durch ein natürliches Protein spezifisch erkannt wird.

2. Mittel nach Anspruch 1, **dadurch gekennzeichnet, dass** es aus einem Antisinn-Oligonukleotid gebildet wird.

3. Mittel nach Anspruch 2, **dadurch gekennzeichnet, dass** es aus einer einzelsträngigen Oligonukleotidsequenz gebildet wird, deren 5'- und 3'-Enden durch eine kovalente Nicht-Nukleotid-Struktur verbunden sind.

4. Mittel nach Anspruch 2, **dadurch gekennzeichnet, dass** es aus einer einzelsträngigen Oligonukleotidsequenz gebildet wird, deren 5'- und 3'-Enden miteinander durch eine Verbindung durch eine Nukleotid-Bindung verbunden sind.

5. Mittel nach einem der Ansprüche 2 bis 4, **dadurch gekennzeichnet, dass** die Oligonukleotidsequenzen keinerlei freies 5'- oder 3'-Ende umfassen.

6. Mittel nach einem der Ansprüche 1 bis 5, das aus einem Antisinn-Oligonukleotid gebildet wird, **dadurch gekennzeichnet, dass** die Nukleotidsequenz(en) der Zusammensetzung keine Fragmente umfassen, die zur Selbstpaarbildung in der Lage sind.

7. Mittel nach einem der Ansprüche 2 bis 6, **dadurch gekennzeichnet, dass** wenigstens eine Oligonukleotidsequenz die zu einer mRNA-Region oder einem Fragment natürlicher DNA komplementäre Sequenz ist.

8. Mittel nach einem der Ansprüche 1 bis 7, **dadurch gekennzeichnet, dass** es einen Polyribonukleotid-Abschnitt umfasst, der in der Lage ist, eine trans-Spaltungsaktivität auf eine RNA auszuüben.

9. Mittel nach Anspruch 1, **dadurch gekennzeichnet, dass** die Oligonukleotidsequenzen Fragmente umfassen, die zu einer Paarbildung unter Bildung von doppelsträngigen Selbstpaarungen in der Lage sind.

10. Mittel nach Anspruch 9, **dadurch gekennzeichnet, dass** die Fragmente, die zu einer Paarbildung in der Lage sind, durch Fragmente, die zu einer Paarbildung nicht in der Lage sind, getrennt sein können.

11. Mittel nach Anspruch 10, **dadurch gekennzeichnet, dass** das natürliche Protein ein Transkriptionsfaktor ist.

12. Mittel nach Anspruch 11, **dadurch gekennzeichnet, dass** der Transkriptionsfaktor HNF-1 ist..

13. Mittel nach Anspruch 12, **dadurch gekennzeichnet, dass** die Fragmente, die zu einer Paarbildung in der Lage sind, durch Fragmente, die zu einer Paarbildung nicht in der Lage sind, getrennt sein können.

14. Mittel nach einem der Ansprüche 2 bis 11, **dadurch gekennzeichnet, dass** die Oligonukleotidsequenz(en) hintereinander angeordnete natürliche oder nicht-natürliche Desoxyribo- oder Ribonukleotide umfassen können.

15. Mittel nach einem der Ansprüche 11 und 14, **dadurch gekennzeichnet, dass** eine Verbindung, die eine biologische Aktivität aufweist, auf die geschlossene einzelsträngige Struktur aufgepfropft ist.

16. Mittel nach Anspruch 15, **dadurch gekennzeichnet, dass** die Verbindung auf die kovalente Nicht-Nukleotid-Struktur aufgepfropft ist.

17. Mittel nach einem der Ansprüche 11, 14-16, **dadurch gekennzeichnet, dass** die kovalente Nicht-Nukleotid-Struktur eine Peptidstruktur ist.

18. Mittel nach einem der Ansprüche 1 bis 11, 14-17, **dadurch gekennzeichnet, dass** die kovalente Nicht-Nukleotid-Struktur eine wenigstens teilweise lipidartige Struktur ist.

19. Mittel nach einem der Ansprüche 1 bis 11, 14-18, **dadurch gekennzeichnet, dass** die Oligonukleotidsequenz ausschließlich aus hintereinander angeordneten natürlichen oder nicht-natürlichen Desoxyribo-Verbindungen oder hintereinander angeordneten natürlichen oder nicht-natürlichen Ribo-Verbindungen besteht.

20. Mittel nach einem der Ansprüche 1 bis 11, 14-19, **dadurch gekennzeichnet, dass** die Nukleotidsequenzen es diesem erlauben, eine doppelsträngige Struktur anzunehmen, innerhalb von welcher antiparallele und/oder parallele Paarungen in einer Konformation vom Moebius-Typ auftreten.

21. Mittel nach einem der Ansprüche 1 bis 11, 14-20, **dadurch gekennzeichnet, dass** die Oligonukleotidsequenzen zwischen 10 und 200 Nukleotiden umfassen.

22. Verfahren zur Herstellung eines Mittels nach einem der Ansprüche 1 bis 21, **dadurch gekennzeichnet, dass** man die partielle oder vollständige chemische Synthese der einzelsträngigen Kette der Verbindung vornimmt, bevor man sie cyclisiert.

23. Verfahren nach Anspruch 22, **dadurch gekennzeichnet, dass** die Oligonukleotidsequenz durch ein biochemisches Verfahren erhalten wird.

24. Mittel nach einem der Ansprüche 1 bis 11, 14-21 für die Herstellung eines Arzneimittels.

25. Verwendung eines Mittels nach einem der Ansprüche 1 bis 11, 14 bis 21 für die Herstellung eines antiviralen oder Antikrebs-Arzneimittels.

26. Mittel nach Anspruch 24, **dadurch gekennzeichnet, dass** das Arzneimittel natürliche immunmodulierende Substanzen induziert.

27. Mittel nach Anspruch 24, **dadurch gekennzeichnet, dass** das Arzneimittel Interferone induziert.

28. Mittel nach einem der Ansprüche 1 bis 11, 14 bis 21 für die Herstellung eines Arzneimittels, das dazu bestimmt ist, auf spezifische Weise auf die Transkription oder die Translation von Proteinen, deren Expressionsniveau man zu modulieren wünscht, einzuwirken.

29. Mittel nach Anspruch 28 als Mittel zur Unterdrückung oder Hemmung der Expression von Proteinen, die direkt an Krankheiten, die mit Proliferation und Differenzierung in Zusammenhang stehen, beteiligt sind.

30. Verwendung eines Mittels nach einem der Ansprüche 1 bis 11, 14 bis 21 für die Herstellung eines Diagnosemittels.

31. Verwendung nach Anspruch 30, **dadurch gekennzeichnet, dass** das Mittel markiert ist.

32. Verwendung eines Mittels nach einem der Ansprüche 1 bis 11, 14 bis 21 in der Kosmetik.

33. Pharmazeutische Zusammensetzung, **dadurch gekennzeichnet, dass** sie als Wirkstoff wenigstens ein Mittel nach einem der Ansprüche 1 bis 11, 14 bis 21 umfasst.

34. Zusammensetzung nach Anspruch 33, **dadurch gekennzeichnet, dass** die pharmazeutische Zusammensetzung in einer auf topischem äußerlichem Wege verabreichbaren Form vorliegt.

35. Zusammensetzung nach Anspruch 34, **dadurch gekennzeichnet, dass** es sich um eine antivirale Zusammensetzung handelt.
